# EUROPÄISCHE PATENTSCHRIFT

(11) **EP 0 770 067 B1**
(45) Veröffentlichungstag und Bekanntmachung des Hinweises auf die Patenterteilung: **13.03.2002**
(21) Anmeldenummer: 95924968.1
(22) Anmeldetag: 04.07.1995
(51) Int. Cl.: C07D 239/54, C07D 209/48, C07D 413/10, A01N 43/54

(54) **SUBSTITUIERTE ZIMTOXIM- UND ZIMTHYDROXAMID-DERIVATE**
SUBSTITUTED CINNAMIC OXIME AND HYDROXAMIDE DERIVATIVES
DERIVES SUBSTITUES D'OXIME CINNAMIQUE ET D'HYDROXAMIDE CINNAMIQUE

(30) Priorität: 14.07.1994 DE 4424791
(43) Veröffentlichungstag der Anmeldung: 02.05.1997
(73) Patentinhaber: BASF AKTIENGESELLSCHAFT, 67056 Ludwigshafen (DE)
(72) Erfinder: KLINTZ, Ralf, D-67269 Grünstadt (DE); HAMPRECHT, Gerhard, D-69469 Weinheim (DE); HEISTRACHER, Elisabeth, D-67061 Ludwigshafen (DE); SCHÄFER, Peter, D-67308 Ottersheim (DE); VON DEM BUSSCHE-HÜNNEFELD, Christoph-Sweder, D-68199 Mannheim (DE); MÜNSTER, Peter, D-67354 Römerberg (DE); KIRSTGEN, Reinhard, D-67434 Neustadt (DE); HARREUS, Albrecht, D-67063 Ludwigshafen (DE); WESTPHALEN, Karl-Otto, D-67346 Speyer (DE); GERBER, Matthias, D-67117 Limburgerhof (DE); WALTER, Helmut, D-67283 Obrigheim (DE)
(86) Internationale Anmeldenummer: EP9502584
(87) Internationale Veröffentlichungsnummer: WO9602518

(56) Entgegenhaltungen:
- EP-A- 0 358 108
- DE-A- 4 131 038
- US-A- 4 979 982
- CHEMICAL ABSTRACTS, vol. 105, no. 7, 1986, Columbus, Ohio, US; abstract no. 60524v, Seite 606 ;Spalte 2 ; in der Anmeldung erwähnt & JP,A,6 127 962 (MITSUBISHI) 7. Februar 1986 in der Anmeldung erwähnt

## Beschreibung

Die vorliegende Erfindung betrifft neue substituierte Zimtoxim-Derivate der Formel I 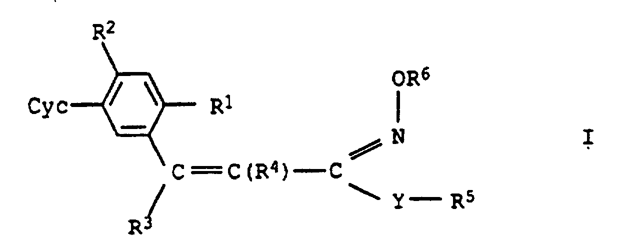 in der die Variablen folgende Bedeutung haben:
- R¹: Halogen, Nitro, Cyano oder Trifluormethyl;
- R²: Wasserstoff oder Halogen;
- R³: Wasserstoff, Halogen, C₁-C₆-Alkyl, C₁-C₆-Halogenalkyl, C₃-C₆-Cycloalkyl, C₃-C₆-Alkenyl, C₃-C₆-Alkinyl oder Hydroxy-C₁-C₆-alkyl;
- R⁴: Wasserstoff, Halogen, Cyano, C₁-C₆-Alkyl, C₁-C₆-Halogenalkyl,
oder R³ und R⁴ zusammen eine chemische Bindung;
- Y: Sauerstoff, Schwefel, Oxycarbonyl, Oxysulfonyl oder eine chemische Bindung;
- R⁵: C₁-C₆-Alkyl oder C₁-C₆-Halogenalkyl, wobei diese Gruppen unsubstituiert sein oder einen der folgenden Reste tragen können: Hydroxyl, Cyano, Hydroxycarbonyl, C₁-C₆-Alkoxy, C₁-C₆-Alkylthio, (C₁-C₆-Alkyl)carbonyl, (C₁-C₆-Alkoxy)carbonyl, (C₁-C₆-Alkyl)carbonyloxy oder einen mit dem Stickstoffatom über eine Carbonylbrücke gebundenen 3- bis 7-gliedrigen Azaheterocyclus, der neben Kohlenstoffringgliedern noch ein Sauerstoff- oder Schwefelatom als Ringglied enthalten kann;
C₃-C₆-Cycloalkyl, C₃-C₆-Alkenyl, C₃-C₆-Alkinyl, Phenyl oder Phenyl-C₁-C₆-alkyl, wobei die Phenylringe gewünschtenfalls ein bis drei Substituenten tragen können, ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
oder, sofern Y Sauerstoff, Schwefel oder eine chemische Bindung bedeutet, (C₁-C₆-Alkyl)carbonyl oder (C₁-C₆-Alkoxy)carbonyl
oder, sofern Y eine chemische Bindung bedeutet, Wasserstoff oder Halogen;
- R⁶: Wasserstoff, C₁-C₆-Alkyl, C₁-C₆-Halogenalkyl, C₃-C₆-Cycloalkyl, C₃-C₆-Alkenyl, C₃-C₆-Alkinyl, Hydroxy-C₁-C₆-alkyl, C₁-C₆-Alkoxy-C₁-C₆-alkyl, C₁-C₆-Alkylthio-C₁-C₆-alkyl, Cyano-C₁-C₆-alkyl, (C₁-C₆-Alkoxy)carbonyl-C₁-C₆-alkyl, (C₁-C₆-Alkyl)carbonyl-C₁-C₆-alkyl, (C₁-C₆-Alkyl)carbonyloxy-C₁-C₆-alkyl oder Phenyl-C₁-C₆-alkyl, wobei der Phenylring gewünschtenfalls ein bis drei Substituenten tragen kann, ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl
oder, sofern Y Sauerstoff oder Schwefel bedeutet, R⁵ und R⁶ zusammen eine C₁-C₃-Alkylenkette, die einen C₁-C₆-Alkyl-Substituenten tragen kann;
Cyc N-(3,4,5,6-Tetrahydrophthalimido) oder einen Rest 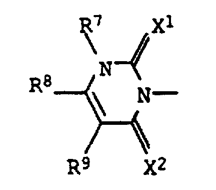 wobei X¹ und X² unabhängig voneinander für Sauerstoff oder Schwefel;
- R⁷: für Wasserstoff, C₁-C₆-Alkyl, C₁-C₆-Halogenalkyl oder Amino und
- R⁸ und R⁹: unabhängig voneinander für Wasserstoff, Halogen, C₁-C₆-Alkyl, C₁-C₆-Halogenalkyl oder Phenyl, das gewünschtenfalls ein bis drei Substituenten tragen kann, ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl stehen;
sowie die landwirtschaftlich brauchbaren Salze der Verbindungen I, sofern diese existieren.

Außerdem betrifft die Erfindung neue substituierte Zimthydroxamid-Derivate der Formel II 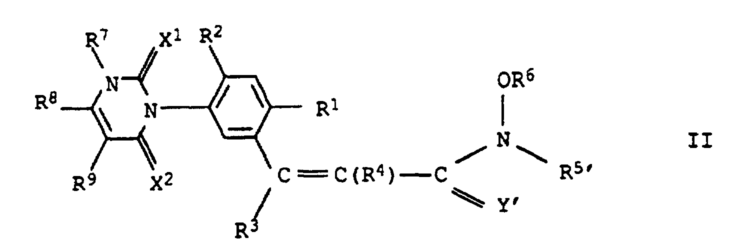 in der die Variablen R¹, R², R³, R⁴, R⁶, R⁷, R⁸, R⁹, X¹ und X² die selben Bedeutungen haben wie bei den Verbindungen der Formel I und wobei
- R⁵': für Wasserstoff, C₁-C₆-Alkyl oder C₁-C₆-Halogenalkyl, wobei die letzten beiden Gruppen einen der folgenden Reste tragen können: Hydroxyl, Cyano, Hydroxycarbonyl, C₁-C₆-Alkoxy, C₁-C₆-Alkylthio, (C₁-C₆-Alkyl)carbonyl (C₁-C₆-Alkoxy)carbonyl, (C₁-C₆-Alkyl)carbonyloxy oder einen mit dem Stickstoffatom über eine Carbonylbrücke gebundenen 3- bis 7-gliedrigen Azaheterocyclus, der neben Kohlenstoffringgliedern noch ein Sauerstoff- oder Schwefelatom als Ringglied enthalten kann;
für C₃-C₆-Cycloalkyl, C₃-C₆-Alkenyl, C₃-C₆-Alkinyl, (C₁-C₆-Alkyl)carbonyl, (C₁-C₆-Halogenalkyl)carbonyl, (C₁-C₆-Alkoxy)carbonyl;
für Phenyl oder Phenyl-C₁-C₆-alkyl, wobei die Phenylringe gewünschtenfalls ein bis drei Substituenten tragen können, ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
und
- Y': für Sauerstoff oder Schwefel stehen.

Des weiteren betrifft die Erfindung
- die Verwendung der Verbindungen I oder II als Herbizide und zur Desiccation und/oder Defoliation von Pflanzen,
- herbizide Mittel und Mittel zur Desiccation und/oder Defoliation von Pflanzen, welche die Verbindungen I oder II als wirksame Substanzen enthalten,
- Verfahren zur Herstellung dieser herbiziden Mittel und Mittel zur Desiccation und/oder Defoliation von Pflanzen,
- Verfahren zur Bekämpfung von unerwünschtem Pflanzenwuchs und zur Desiccation und/oder Defoliation von Pflanzen mit den Verbindungen I oder II, sowie
- neue Zwischenprodukte der Formel IX zur Herstellung der Verbindungen I.

Aus der EP-A-0 385 231 ist bekannt, daß u.a. Verbindungen der Formel IIIa 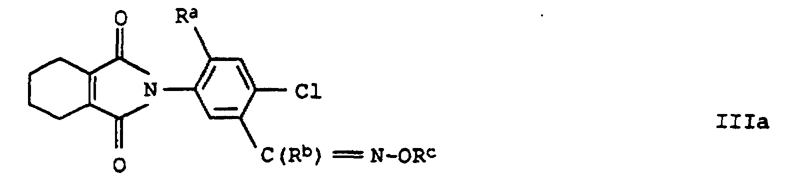 in der R^{a} für Wasserstoff, Fluor oder Chlor, R^{b} für Wasserstoff oder Cyano und R^{c} für C₁-C₆-Alkyl, C₃-C₄-Alkenyl, C₃-C₄-Alkinyl, -CH₂-COOH, -CH(CH₃)-COOH, -CH₂-C(CH₃)₂-COOH, -CH₂-Ester, -CH(CH₃)-Ester oder -CH₂-C(CH₃)₂-Ester stehen, zur Desiccation und Abszission von Pflanzenteilen geeignet sind.

Außerdem wird in der JP-A 61/027 962 gelehrt, daß Verbindungen der Formel IIIb 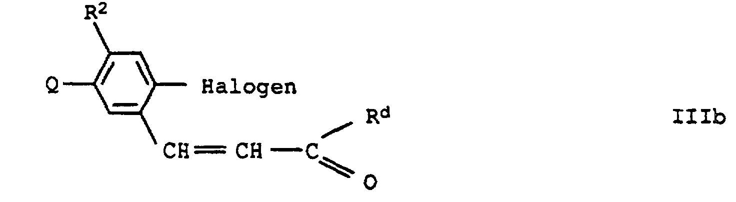 in der Q für N-3,4,5,6-Tetrahydrophthalimido- oder 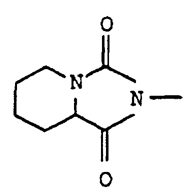 und R^{d} u.a. für die Aminogruppe, die bestimmte Substituenten tragen kann, stehen, herbizid wirksam sind.

In der EP-A-0 358 108 werden Verbindungen der Formel IIIc beschrieben 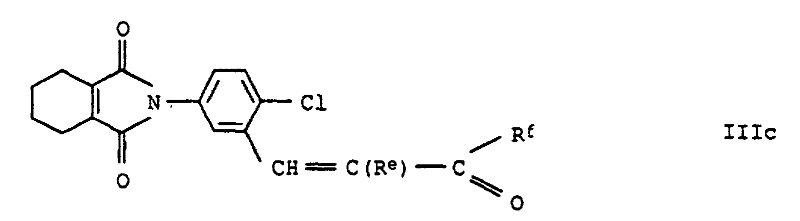 in der R^{e} für Chlor, Brom oder C₁-C₄-Alkyl und R^{f} u.a. für eine ein oder zweifach substituierte Aminogruppe stehen.

Außerdem ist der U.S. 5,035,740 zu entnehmen, daß bestimmte N-Phenyl-Azaheterocyclen, deren Phenylring in 2-Stellung zum Heterocyclus u.a. einen Rest R², in 4-Stellung zum Heterocyclus u.a. ein Wasserstoff- oder Halogenatom oder einen Halogenalkylrest, und in 5-Position zum Heterocyclus u.a. eine gegebenenfalls substituierte 2-(Aminocarbonyl)-ethenylgruppe trägt, herbizid wirksam sind.

In der EP-A-0 379 911 werden N-Phenyltetrahydroindazol-Derivate der Formel IIId 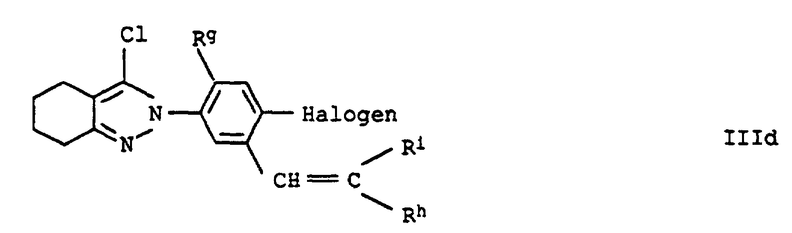 in der R^{g} für Wasserstoff oder Fluor, R^{h} für Wasserstoff, Halogen oder C₁-C₄-Alkyl und Rⁱ u.a. für einen Säureamid-Rest stehen, als Herbizide beschrieben.

Aus der WO 90/02120 ist bekannt, daß bestimmte 1-Phenyl-4,5-dihydro-1,2,4-triazol-5-(1H)-one, deren Phenylring in meta-Stellung zum Heterocyclus u.a. eine gegebenenfalls substituierte 2-(Aminocarbonyl)ethenylgruppe trägt, herbizide Wirkung zeigen.

Außerdem sind aus der U.S. 4,979,982 herbizid wirksame 3-Phenylurazile der Formel IIIe bekannt 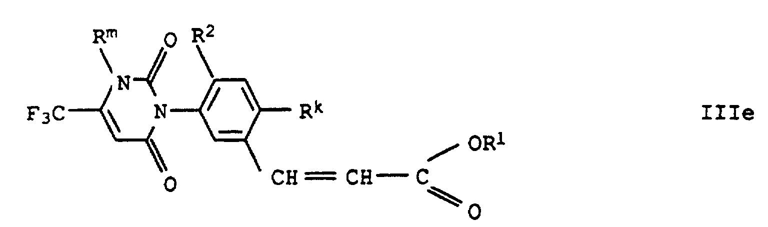 wobei R^{k} Wasserstoff oder Halogen, R^{l} C₁-C₁₂-Alkyl oder Cycloalkyl und R^{m} C₁-C₁₂-Alkyl oder C₃-C₁₂-Alkenyl bedeuten.

Den Verbindungen IIIe strukturell ähnliche Herbizide werden außerdem in der WO 93/06090 beschrieben.

Der EP-A 408 382 ist zu entnehmen, daß u.a. Uracil-Derivate der Formel IIIf 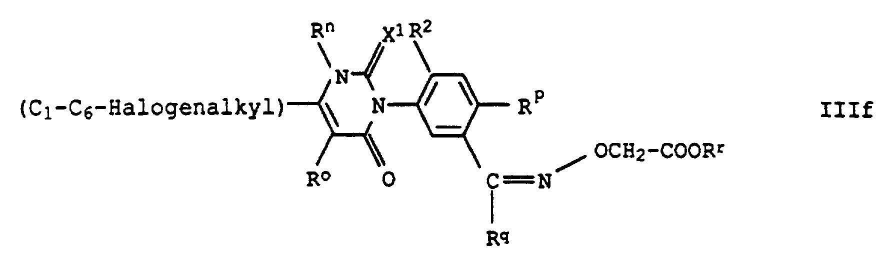 wobei Rⁿ Wasserstoff, Hydroxymethyl oder C₁-C₃-[Halogen-]Alkyl, R^{o} Wasserstoff, Nitro, Halogen, C₁-C₆-[Halogen-]Alkyl oder Hydroxymethyl, R^{p} Nitro, Cyano oder Halogen, R^{q} Wasserstoff, C₁-C₃-Alkyl, -Alkoxy oder -Alkoxy-C₁-C₂-alkyl und R^{r} Wasserstoff, C₁-C₄-Alkyl, C₂-C₃-Halogenalkyl, C₃-C₆-Cycloalkyl, Phenyl oder Benzyl bedeuten, eine herbizide Wirkung aufweisen.

Gemäß der WO 89/02891 sind bestimmte 3-(3-Aminocarbonyl-phenyl)uracile ebenfalls herbizid wirksam.

Schließlich wird in der WO 93/11669 gelehrt, daß bestimmte 3-(3-Aminocarbonyl)urazile zur Desikkation und Abszission von Pflanzenorganen geeignet sind.

Da die Selektivität der bekannten Herbizide bezüglich der Schadpflanzen nur bedingt zu befriedigen vermag, lagen der Erfindung neue herbizid wirksame Verbindungen als Aufgabe zugrunde, mit denen sich - bei guter Verträglichkeit für die Nutzpflanzen - die Schadpflanzen besser als bisher gezielt bekämpfen lassen.

Demgemäß wurden die substituierten Zimtoxim-Derivate der Formel I gefunden. Außerdem wurden neue Verbindungen der Formel II gefunden, die wertvolle Zwischenprodukte zur Synthese der Verbindungen I sind oder bei deren Herstellung als Nebenprodukte anfallen.

Des weiteren wurden herbizide Mittel gefunden, die die Verbindungen I oder II enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I oder II gefunden.

Die erfindungsgemäßen Verbindungen I und II eignen sich des weiteren zur Defoliation und Desikkation von Pflanzenteilen für z.B. Baumwolle, Kartoffel, Raps, Sonnenblume, Sojabohne oder Ackerbohnen, insbesondere für Baumwolle. Diesbezüglich wurden Mittel zur Desikkation und/oder Defoliation von Pflanzen, Verfahren zur Herstellung dieser Mittel und Verfahren zur Desiccation und/oder Defoliation von Pflanzen mit den Verbindungen I oder II gefunden.

Die für die Substituenten R¹ bis R⁹ oder als Reste an Phenylringen oder Heterocyclen genannten organischen Molekülteile stellen - wie die Bedeutung Halogen- Sammelbegriffe für individuelle Aufzählungen der einzelnen Gruppenmitglieder dar. Sämtliche Kohlenstoffketten, also alle Alkyl-, Halogenalkyl, Alkenyl-, Alkinyl-, Hydroxyalkyl-, Cyanoalkyl, Alkoxy-, Alkylthio-, Alkylcarbonyl-, Halogenalkylcarbonyl-, Alkoxycarbonyl-, Alkoxycarbonylalkyl-, Alkylcarbonyloxy-, Alkylcarbonylalkyl-, Alkylcarbonyloxyalkyl-, Phenylalkyl, Alkoxyalkyl- und Alkylthioalkyl-Teile können geradkettig oder verzweigt sein. Halogenierte Substituenten tragen vorzugsweise ein bis fünf gleiche oder verschiedene Halogenatome.

Im einzelnen stehen beispielsweise:
- Halogen für: Fluor, Chlor, Brom und Jod, vorzugsweise für Fluor und Chlor;
- C₁-C₆-Alkyl und die Alkyl-Teile von C₁-C₆-Alkoxy-C₁-C₆-alkyl, C₁-C₆-Alkylthio-C₁-C₆-alkyl, (C₁-C₆-Alkyl)carbonyl-C₁-C₆-alkyl, (C₁-C₆-Alkoxy)carbonyl-C₁-C₆-alkyl, (C₁-C₆-Alkyl)carbonyloxy-C₁-C₆-alkyl und Phenyl-C₁-C₆-alkyl für: Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise für C₁-C₄-Alkyl, insbesondere für Methyl und Ethyl;
- C₁-C₆-Halogenalkyl für: C₁-C₆-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Fluorpropyl, 3-Chlorpropyl und Heptafluorpropyl, vorzugsweise für C₁-C₄-Halogenalkyl, insbesondere für Trifluormethyl und 1,2-Dichlorethyl;

- C₃-C₆-Alkenyl für: Prop-1-en-1-yl, Prop-2-en-1-yl, 1-Methyl-ethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2, 3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl, vorzugsweise für C₃- oder C₄-Alkenyl;
- C₃-C₆-Alkinyl für: Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise für C₃- oder C₄-Alkinyl, insbesondere für Prop-2-in-3-yl;
- Hydroxy-C₁-C₆-alkyl z.B. für: Hydroxymethyl, 1-Hydroxy-eth-1-yl, 2-Hydroxyeth-1-yl, 1-Hydroxyprop-1-yl, 2-Hydroxy-prop-1-yl, 3-Hydroxyprop-1-yl, 1-Hydroxyprop-2-yl, 2-Hydroxy-prop-2-yl, 1-Hydroxybut-1-yl, 2-Hydroxybut-1-yl, 3-Hydroxy-but-1-yl, 4-Hydroxybut-1-yl, 1-Hydroxybut-2-yl, 2-Hydroxy-but-2-yl, 1-Hydroxybut-3-yl, 2-Hydroxybut-3-yl, 1-Hydroxy-2-methyl-prop-3-yl, 2-Hydroxy-2-methyl-prop-3-yl, 3-Hydroxy-2-methyl-prop-3-yl und 2-Hydroxymethyl-prop-2-yl, vorzugsweise für Hydroxy-C₁-C₆-alkyl, insbesondere für 2-Hydroxyeth-1-yl;
- Cyano-C₁-C₆-alkyl z.B. für: Cyanomethyl, 1-Cyanoeth-1-yl, 2-Cyanoeth-1-yl, 1-Cyanoprop-1-yl, 2-Cyanoprop-1-yl, 3-Cyano-prop-1-yl, 1-Cyanoprop-2-yl, 2-Cyanoprop-2-yl, 1-Cyano-but-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyano-but-3-yl, 1-Cyano-2-methyl-prop-3-yl, 2-Cyano-2-methyl-prop-3-yl, 3-Cyano-2-methyl-prop-3-yl und 2-Cyanomethyl-prop-2-yl, vorzugsweise für Cyano-C₁-C₄-alkyl, insbesondere für 2-Cyanoeth-1-yl;
- Phenyl-C₁-C₆-alkyl z.B. für: Benzyl, 1-Phenyleth-1-yl, 2-Phenyleth-1-yl, 1-Phenylprop-1-yl, 2-Phenylprop-1-yl, 3-Phenylprop-1-yl, 1-Phenylprop-2-yl, 2-Phenylprop-2-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 1-Phenylbut-3-yl, 2-Phenylbut-3-yl, 1-Phenyl-2-methyl-prop-3-yl, 2-Phenyl-2-methyl-prop-3-yl, 3-Phenyl-2-methyl-prop-3-yl und 2-Benzyl-prop-2-yl, vorzugsweise für Phenyl-C₁-C₄-alkyl, insbesondere für 2-Phenyleth-1-yl;
- C₁-C₆-Alkoxy und der Alkoxy-Teil von C₁-C₆-Alkoxy-C₁-C₆-alkyl für: Methoxy, Ethoxy, n-Propoxy, 1-Methylethoxy, n-Butoxy, 1-Methyl-propoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, vorzugsweise für C₁-C₄-Alkoxy, insbesondere für Methoxy, Ethoxy und 1-Methylethoxy;
- C₁-C₆-Alkylthio für: Methylthio, Ethylthio, n-Propylthio, 1-Methylethylthio, n-Butylthio, 1-Methylpropylthio, 2-Methylpropylthio, 1,1-Dimethylethylthio, n-Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, n-Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio', 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, vorzugsweise für C₁-C₄-Alkylthio, insbesondere für Methylthio, Ethylthio und 1-Methylethylthio;
- (C₁-C₆-Alkyl)carbonyl und der Alkylcarbonyl-Teil von (C₁-C₆-Alkyl)carbonyl-C₁-C₆-alkyl für: Methylcarbonyl, Ethylcarbonyl, n-Propylcarbonyl, 1-Methylethyl-carbonyl, n-Butylcarbonyl, 1-Methylpropylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, n-Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise für (C₁-C₄-Alkyl)carbonyl, insbesondere für Methylcarbonyl und Ethylcarbonyl;
- (C₁-C₆-Halogenalkyl)carbonyl für: (C₁-C₆-Alkyl)carbonyl wie vorstehend genannt, das partiell oder vollständig durch Fluor, Chlor und/oder Brom substituiert ist, also z.B. Chlormethylcarbonyl, Dichlormethylcarbonyl, Trichlormethylcarbonyl, Fluormethylcarbonyl, Difluormethylcarbonyl, Trifluormethylcarbonyl, Chlorfluormethylcarbonyl, Dichlorfluormethylcarbonyl, Chlordifluormethylcarbonyl, 1-Fluorethylcarbonyl, 2-Fluorethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 3-Chlorpropylcarbonyl, Heptafluorpropylcarbonyl, vorzugsweise für (C₁-C₄-Halogenalkyl)carbonyl, insbesondere für Trifluormethylcarbonyl, Chlormethylcarbonyl, Dichlormethylcarbonyl und Trichlormethylcarbonyl;
- (C₁-C₆-Alkoxy)carbonyl und der Alkoxycarbonyl-Teil von (C₁-C₆-Alkoxy)carbonyl-C₁-C₆-alkyl für: Methoxycarbonyl, Ethoxycarbonyl, n-Propoxycarbonyl, 1-Methyl-ethoxycarbonyl, n-Butoxycarbonyl, 1-Methylpropoxycarbonyl, 2-Methylpropoxycarbonyl, 1,1-Dimethylethoxycarbonyl, n-Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, n-Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl und 1-Ethyl-2-methyl-propoxycarbonyl, vorzugsweise für (C₁-C₄-Alkoxy)carbonyl, insbesondere für Methoxycarbonyl, Ethoxycarbonyl und 1-Methylethoxycarbonyl;
- der Alkylcarbonyloxy-Teil von (C₁-C₆-Alkyl)carbonyloxy-C₁-C₆-alkyl für: Methylcarbonyloxy, Ethylcarbonyloxy, n-Propylcarbonyloxy, (1-Methylethyl)carbonyloxy, n-Butylcarbonyloxy, (1-Methylpropyl)carbonyloxy, (2-Methylpropyl)carbonyloxy, (1,1-Dimethylethyl)carbonyloxy, n-Pentyl-carbonyloxy, (1-Methylbutyl)carbonyloxy, (2-Methylbutyl)carbonyloxy, (3-Methylbutyl)carbonyloxy, (2,2-Dimethylpropyl)carbonyloxy, (1-Ethylpropyl)carbonyloxy, n-Hexylcarbonyloxy, (1,1-Dimethylpropyl)carbonyloxy, (1,2-Dimethylpropyl)carbonyloxy, (1-Methylpentyl)carbonyloxy, (2-Methylpentyl)carbonyloxy, (3-Methylpentyl)carbonyloxy, (4-Methylpentyl)carbonyloxy, (1,1-Dimethylbutyl)carbonyloxy, (1,2-Dimethylbutyl)carbonyloxy, (1,3-Dimethylbutyl)carbonyloxy, (2,2-Dimethylbutyl)carbonyloxy, (2,3-Dimethylbutyl)carbonyloxy, (3,3-Dimethylbutyl)carbonyloxy, (1-Ethylbutyl)carbonyloxy, (2-Ethylbutyl)carbonyloxy, (1,1,2-Trimethylpropyl)carbonyloxy, (1,2,2-Trimethylpropyl)carbonyloxy, (1-Ethyl-1-methylpropyl)carbonyloxy und (1-Ethyl-2-methylpropyl)carbonyloxy, vorzugsweise für (C₁-C₄-Alkyl)carbonyloxy, insbesondere für Methylcarbonyloxy und Ethylcarbonyloxy;
- C₃-C₆-Cycloalkyl für: Cyclopropyl, Cyclobutyl, Cyclopentyl und Cyclohexyl, vorzugsweise für Cyclopropyl und Cyclopentyl;
3- bis 7-gliedrige Azaheterocyclen, die neben Kohlenstoffringgliedern noch ein Sauerstoff- oder Schwefelatom als Ringglied enthalten können, sind z.B.
Pyrrolidin-1-yl, Isoxazolidin-2-yl, Isothiazolidin-2-yl, Oxazolidin-3-yl, Thiazolidin-3-yl, Piperidin-1-yl, Azepin-1-yl, Morpholin-1-yl und Thiomorpholin-1-yl.

Alle Phenylringe sind vorzugsweise unsubstituiert oder tragen einen Halogen-, Methyl-, Trifluormethyl- oder Methoxy-Substituenten.

In Abhängigkeit von den jeweiligen Substituenten können die Verbindungen I und II in Form ihrer landwirtschaftlich brauchbaren Salze vorliegen, wobei es auf die Art des Salzes im allgemeinen nicht ankommt. Üblicherweise werden Salze von solchen Basen oder solchen Säuren in Betracht kommen, welche die herbizide Wirkung von I oder II nicht negativ beeinträchtigen.

Als basische Salze eignen sich besonders diejenigen der Alkalimetalle, vorzugsweise die Natrium- und Kaliumsalze, die der Erdalkalimetalle, vorzugsweise Calcium-, Magnesium- und Bariumsalze, und die der Übergangsmetalle, vorzugsweise Mangan-, Kupfer-, Zink- und Eisensalze, sowie Ammoniumsalze, bei denen das Ammoniumion gewünschtenfalls ein bis drei C₁-C₄-Alkyl-, Hydroxy-C₁-C₄-alkylsubstituenten und/oder einen Phenyl- oder Benzylsubstituenten tragen kann, vorzugsweise Diisopropylammonium-, Tetramethylammonium-, Tetrabutylammonium-, Trimethylbenzylammonium und Trimethyl-(2-hydroxyethyl)-ammoniumsalze, des weiteren Phosphoniumsalze, Sulfoniumsalze wie vorzugsweise Tri-(C₁-C₄-alkyl)sulfoniumsalze, und Sulfoxoniumsalze wie vorzugsweise Tri-(C₁-C₄-alkyl)sulfoxoniumsalze.

Säureadditionssalze sind beispielsweise die Hydrochloride und -bromide, Sulfate, Nitrate, Phosphate, Oxalate oder die Dodecylbenzolsulfonate von Verbindungen I.

Im Hinblick auf die Verwendung der substituierten Zimtoxim-Derivate I und der substituierten Zimthydroxamid-Derivate II als Herbizide sind diejenigen Verbindungen bevorzugt, in denen die Variablen folgende Bedeutungen haben, und zwar jeweils für sich allein oder in Kombination:
- R¹: Halogen oder Cyano, insbesondere für Chlor, Brom oder Cyano;
- R²: Wasserstoff, Fluor oder Chlor;
- R³: Wasserstoff oder Halogen;
- R⁴: Wasserstoff, Halogen oder C₁-C₄-Alkyl;
- Y: Sauerstoff oder eine chemische Bindung;
- Y': Sauerstoff;
- R⁵: C₁-C₄-Alkyl, C₁-C₆-Alkoxy-C₁-C₆-alkyl, (C₁-C₆-Alkoxy)carbonyl-C₁-C₆-alkyl oder, sofern Y eine chemische Bindung bedeutet, Wasserstoff;
- R⁵': Wasserstoff oder C₁-C₄-Alkyl;
- R⁶: Wasserstoff, C₁-C₄-Alkyl oder C₃-C₄-Alkenyl;
Cyc einen Rest 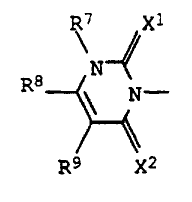 wobei X¹ und X² unabhängig voneinander für Sauerstoff oder Schwefel;
- R⁷: für Methyl oder Amino und
- R⁸: für C₁-C₄-Halogenalkyl, insbesondere für Trifluormethyl oder Chlordifluormethyl und
- R⁹: für Wasserstoff
stehen.

Besonders bevorzugt sind die substituierten Zimtoxim-Derivate I der folgenden Tabellen 1 und 2:

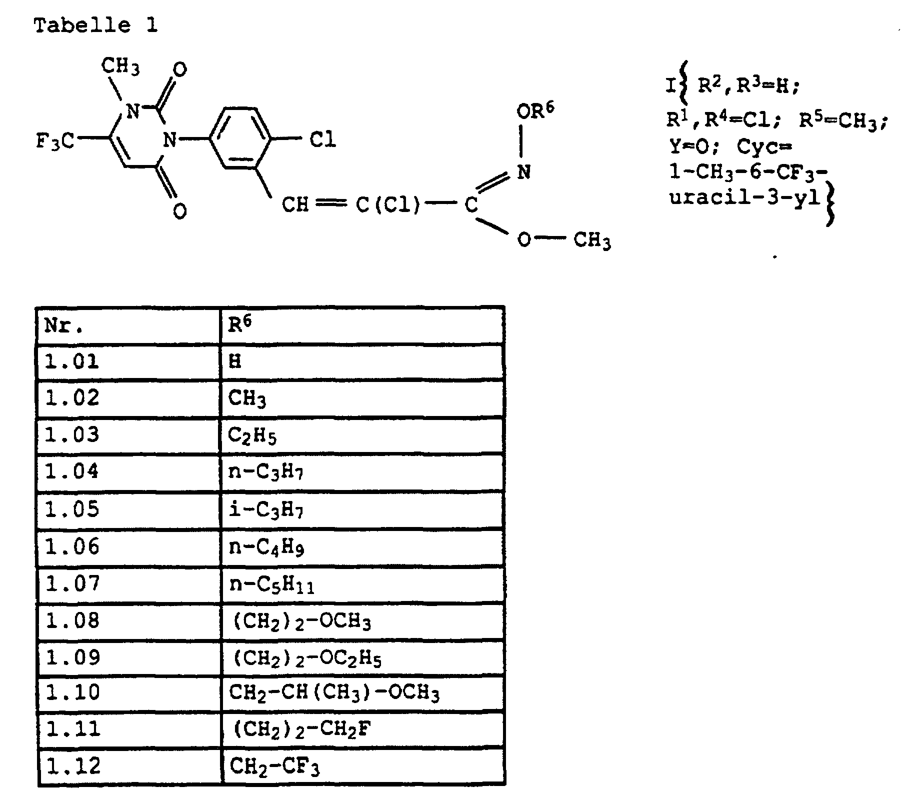

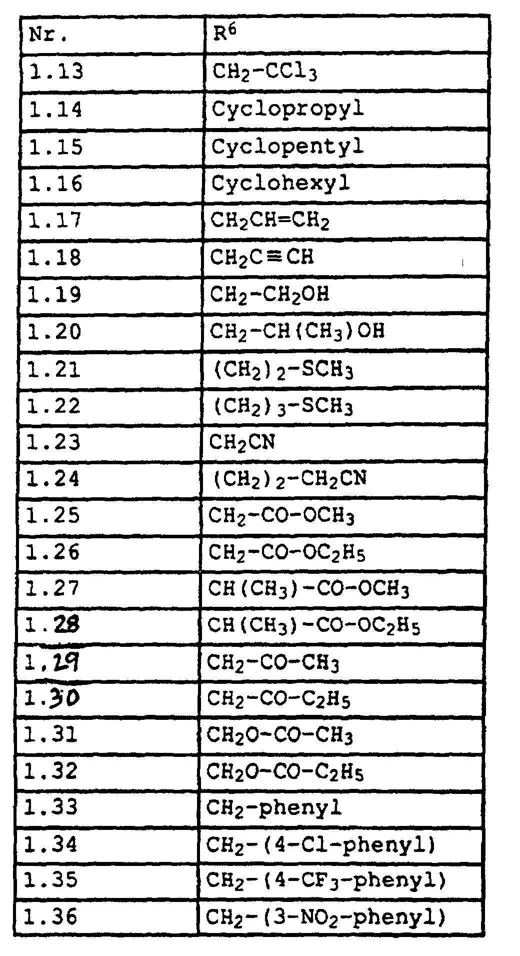

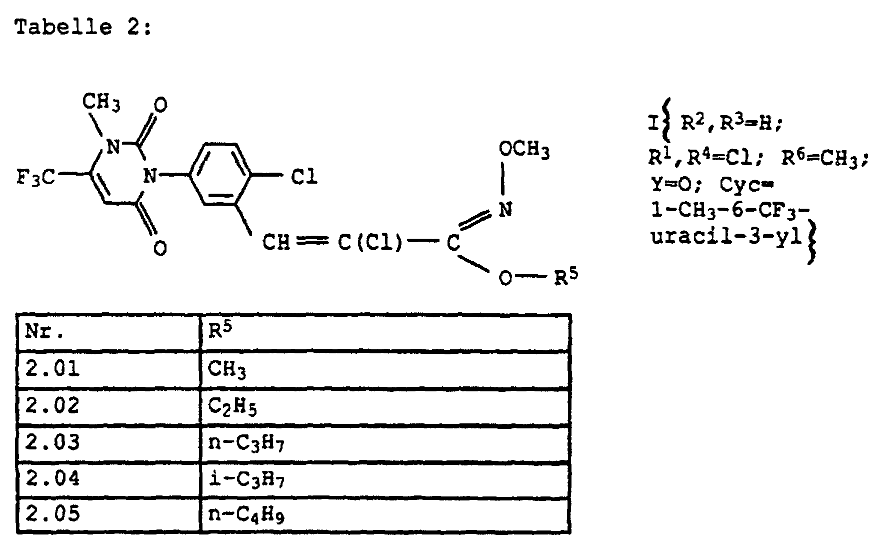

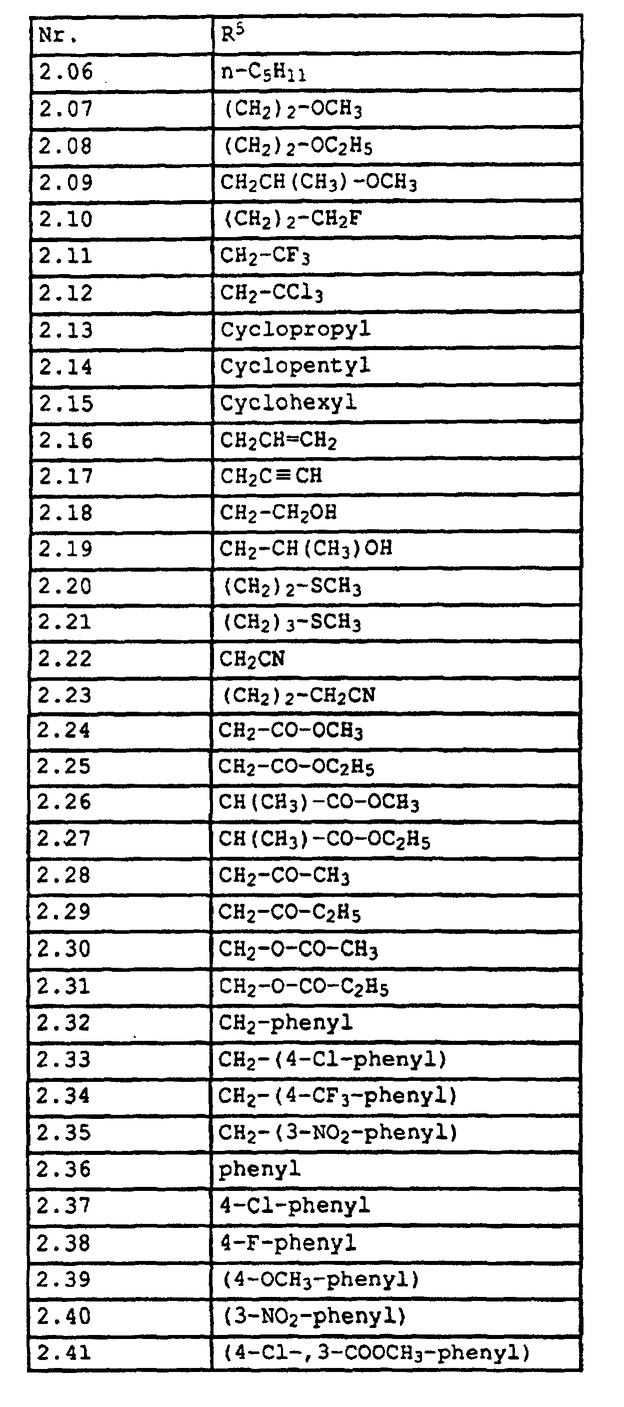

Des weiteren sind die folgenden substituierten Zimtoxim-Derivate I besonders bevorzugt:
- die Verbindungen 3.01 - 3.36, die sich von den Verbindungen 1.01 - 1.36 dadurch unterscheiden, daß R² Fluor bedeutet;
- die Verbindungen 4.01 - 4.36, die sich von den Verbindungen 1.01 - 1.36 dadurch unterscheiden, daß R⁴ Brom bedeutet;
- die Verbindungen 5.01 - 5.36, die sich von den Verbindungen 1.01 - 1.36 dadurch unterscheiden, daß R⁴ Methyl bedeutet;
- die Verbindungen 6.01 - 6.36, die sich von den Verbindungen 1.01 - 1.36 dadurch unterscheiden, daß R⁴ Wasserstoff bedeutet;
- die Verbindungen 7.01 - 7.36, die sich von den Verbindungen 1.01 - 1.36 dadurch unterscheiden, daß R² Fluor und R⁴ Brom bedeuten;
- die Verbindungen 8.01 - 8.36, die sich von den Verbindungen 1.01 - 1.36 dadurch unterscheiden, daß R² Fluor und R⁴ Methyl bedeuten;
- die Verbindungen 9.01 - 9.36, die sich von den Verbindungen 1.01 - 1.36 dadurch unterscheiden, daß R² Fluor und R⁴ Wasserstoff bedeuten;
- die Verbindungen 10.01 - 10.36, die sich von den Verbindungen 1.01 - 1.36 dadurch unterscheiden, daß R² Fluor und Y Schwefel bedeuten;
- die Verbindungen 11.01 - 11.36, die sich von den Verbindungen 1.01 - 1.36 dadurch unterscheiden, daß R⁴ Brom und Y Schwefel bedeuten;
- die Verbindungen 12.01 - 12.36, die sich von den Verbindungen 1.01 - 1.36 dadurch unterscheiden, daß R⁴ Methyl und Y Schwefel bedeuten;
- die Verbindungen 13.01 - 13.36, die sich von den Verbindungen 1.01 - 1.36 dadurch unterscheiden, daß R⁴ Wasserstoff und Y Schwefel bedeuten;
- die Verbindungen 14.01 - 14.36, die sich von den Verbindungen 1.01 - 1.36 dadurch unterscheiden, daß R² Fluor, R⁴ Brom und Y Schwefel bedeuten;
- die Verbindungen 15.01 - 15.36, die sich von den Verbindungen 1.01 - 1.36 dadurch unterscheiden, daß R² Fluor, R⁴ Methyl und Y Schwefel bedeuten;
- die Verbindungen 16.01 - 16.36, die sich von den Verbindungen 1.01 - 1.36 dadurch unterscheiden, daß R² Fluor, R⁴ Wasserstoff und Y eine chemische Bindung bedeuten;
- die Verbindungen 17.01 - 17.36, die sich von den Verbindungen 1.01 - 1.36 dadurch unterscheiden, daß R² Fluor und Y eine chemische Bindung bedeuten;
- die Verbindungen 18.01 - 18.36, die sich von den Verbindungen 1.01 - 1.36 dadurch unterscheiden, daß R⁴ Brom und Y Bindung bedeuten;
- die Verbindungen 19.01 - 19.36, die sich von den Verbindungen 1.01 - 1.36 dadurch unterscheiden, daß R⁴ Methyl und Y eine chemische Bindung bedeuten;
- die Verbindungen 20.01 - 20.36, die sich von den Verbindungen 1.01 - 1.36 dadurch unterscheiden, daß R⁴ Wasserstoff und Y eine chemische Bindung bedeuten;
- die Verbindungen 21.01 - 21.36, die sich von den Verbindungen 1.01 - 1.36 dadurch unterscheiden, daß R² Fluor, R⁴ Brom, Y eine chemische Bindung bedeuten;
- die Verbindungen 22.01 - 22.36, die sich von den Verbindungen 1.01 - 1.36 dadurch unterscheiden, daß R² Fluor, R⁴ Methyl, Y eine chemische Bindung bedeuten;
- die Verbindungen 23.01 - 23.36, die sich von den Verbindungen 1.01 - 1.36 dadurch unterscheiden, daß R² Fluor, R⁴ Wasserstoff, Y eine chemische Bindung und bedeuten;
- die Verbindungen 24.01 - 24.41, die sich von den Verbindungen 2.01 - 2.41 dadurch unterscheiden, daß R² Fluor bedeutet;
- die Verbindungen 25.01 - 25.41, die sich von den Verbindungen 2.01 - 2.41 dadurch unterscheiden, daß R⁴ Brom bedeuten.
- die Verbindungen 26.01 - 26.41, die sich von den Verbindungen 2.01 - 2.41 dadurch unterscheiden, daß R⁴ Methyl bedeutet;
- die Verbindungen 27.01 - 27.41, die sich von den Verbindungen 2.01 - 2.41 dadurch unterscheiden, daß R⁴ Wasserstoff bedeutet;
- die Verbindungen 28.01 - 28.41, die sich von den Verbindungen 2.01 - 2.41 dadurch unterscheiden, daß R⁴ Brom und R² Fluor bedeuten;
- die Verbindungen 29.01 - 29.41, die sich von den Verbindungen 2.01 - 2.41 dadurch unterscheiden, daß R⁴ Methyl und R² Fluor bedeuten;
- die Verbindungen 30.01 - 30.41, die sich von den Verbindungen 2.01 - 2.41 dadurch unterscheiden, daß R⁴ Wasserstoff und R² Fluor bedeuten;
- die Verbindungen 31.01 - 31.41, die sich von den Verbindungen 2.01 - 2.41 dadurch unterscheiden, daß R² Fluor und Y Schwefel bedeuten;
- die Verbindungen 32.01 - 32.41, die sich von den Verbindungen 2.01 - 2.41 dadurch unterscheiden, daß R⁴ Brom und Y Schwefel bedeuten;
- die Verbindungen 33.01 - 33.41, die sich von den Verbindungen 2.01 - 2.41 dadurch unterscheiden, daß R⁴ Methyl und Y Schwefel bedeuten;
- die Verbindungen 34.01 - 34.41, die sich von den Verbindungen 2.01 - 2.41 dadurch unterscheiden, daß R⁴ Wasserstoff und Y Schwefel bedeuten;
- die Verbindungen 35.01 - 35.41, die sich von den Verbindungen 2.01 - 2.41 dadurch unterscheiden, daß R² Fluor, R⁴ Brom und Y Schwefel bedeuten;
- die Verbindungen 36.01 - 36.41, die sich von den Verbindungen 2.01 - 2.41 dadurch unterscheiden, daß R² Fluor, R⁴ Methyl und Y Schwefel bedeuten;
- die Verbindungen 37.01 - 37.41, die sich von den Verbindungen 2.01 - 2.41 dadurch unterscheiden, daß R² Fluor, R⁴ Wasserstoff und Y Schwefel bedeuten;
- die Verbindungen 38.01 - 38.41, die sich von den Verbindungen 2.01 - 2.41 dadurch unterscheiden, daß R² Fluor und Y eine chemische Bindung bedeuten;
- die Verbindungen 39.01 - 39.41, die sich von den Verbindungen 2.01 - 2.41 dadurch unterscheiden, daß R⁴ Brom und Y eine chemische Bindung bedeuten;
- die Verbindungen 40.01 - 40.41, die sich von den Verbindungen 2.01 - 2.41 dadurch unterscheiden, daß R⁴ Methyl und Y eine chemische Bindung bedeuten;
- die Verbindungen 41.01 - 41.41, die sich von den Verbindungen 2.01 - 2.41 dadurch unterscheiden, daß R⁴ Wasserstoff und Y eine chemische Bindung bedeuten;
- die Verbindungen 42.01 - 42.41, die sich von den Verbindungen 2.01 - 2.41 dadurch unterscheiden, daß R² Fluor, R⁴ Brom und Y eine chemische Bindung bedeuten;
- die Verbindungen 43.01 - 43.41, die sich von den Verbindungen 2.01 - 2.41 dadurch unterscheiden, daß R² Fluor, R⁴ Methyl und Y eine chemische Bindung bedeuten;
- die Verbindungen 44.01 - 44.41, die sich von den Verbindungen 2.01 - 2.41 dadurch unterscheiden, daß R² Fluor, R⁴ Wasserstoff und Y eine chemische Bindung bedeuten;
- die Verbindungen 45.01 - 45.36, die sich von den Verbindungen 1.01 - 1.36 dadurch unterscheiden, daß R² Fluor und Cyc 1-NH₂-6-CF₃-uracil-3-yl bedeuten;
- die Verbindungen 46.01 - 46.36, die sich von den Verbindungen 1.01 - 1.36 dadurch unterscheiden, daß R⁴ Brom und Cyc 1-NH₂-6-CF₃-uracil-3-yl bedeuten;
- die Verbindungen 47.01 - 47.36, die sich von den Verbindungen 1.01 - 1.36 dadurch unterscheiden, daß R⁴ Methyl und Cyc 1-NH₂-6-CF₃-uracil-3-yl bedeuten;
- die Verbindungen 48.01 - 48.36, die sich von den Verbindungen 1.01 - 1.36 dadurch unterscheiden, daß R⁴ Wasserstoff und Cyc 1-NH₂-6-CF₃-uracil-3-yl bedeuten;
- die Verbindungen 49.01 - 49.36, die sich von den Verbindungen 1.01 - 1.36 dadurch unterscheiden, daß R² Fluor, R⁴ Brom und Cyc 1-NH₂-6-CF₃-uracil-3-yl bedeuten;
- die Verbindungen 50.01 - 50.36, die sich von den Verbindungen 1.01 - 1.36 dadurch unterscheiden, daß R² Fluor, R⁴ Methyl und Cyc 1-NH₂-6-CF₃-uracil-3-yl bedeuten;
- die Verbindungen 51.01 - 51.36, die sich von den Verbindungen 1.01 - 1.36 dadurch unterscheiden, daß R² Fluor, R⁴ Wasserstoff und Cyc 1-NH₂-6-CF₃-uracil-3-yl bedeuten.

Die substituierten Zimtoxim-Derivate der Formel I sowie die substituierten Zimthydroxamid-Derivate der Formel II sind auf verschiedene Weise erhältlich, vorzugsweise nach einem der folgenden Verfahren:
a) Alkylierung eines subst. Zimthydroxamid-Derivats der Formel II mit R⁵' = Wasserstoff oder der Formel IV: 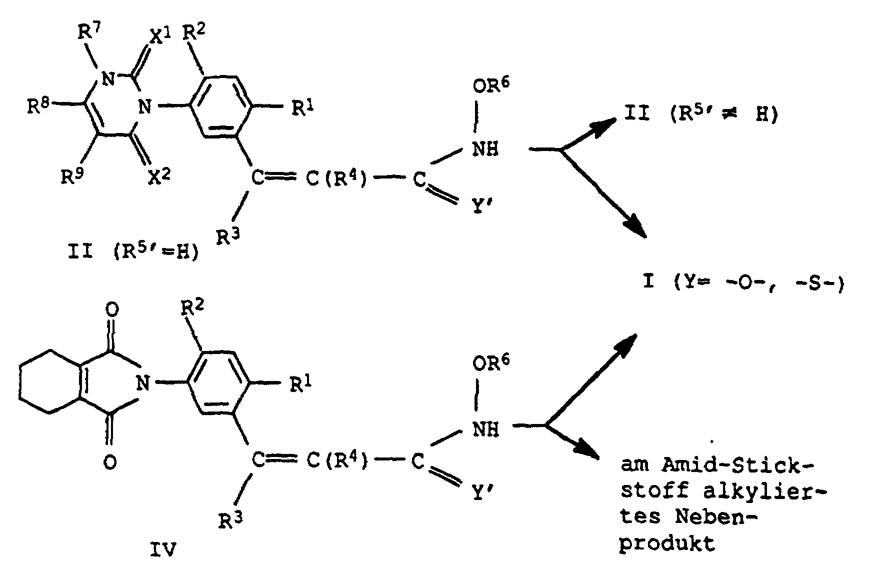
   In der Regel arbeitet man in einem inerten Lösungs- oder Verdünnungsmittel, vorzugsweise in Gegenwart einer Base.
   Als Lösungsmittel eignen sich beispielsweise protische Lösungsmittel wie die niederen Alkohole, vorzugsweise Ethanol, gewünschtenfalls im Gemisch mit Wasser, oder aprotische Lösungsmittel wie aliphatische oder cyclische Ether, vorzugsweise 1,2-Dimethoxyethan, Tetrahydrofuran und Dioxan, aliphatische Ketone, vorzugsweise Aceton, Amide, vorzugsweise Dimethylformamid, Sulfoxide, vorzugsweise Dimethylsulfoxid, Harnstoffe wie Tetramethylharnstoff und 1,3-Dimethyltetrahydro-2(1H)-pyrimidinon, (DMPU) Carbonsäureester wie Essigsäurethylester, oder halogenierte aliphatische oder aromatische Kohlenwasserstoffe wie Dichlormethan und Chlorbenzol.
   Die Alkylierung erfolgt normalerweise mit dem Halogenid, vorzugsweise dem Chlorid oder Bromid, dem Sulfat, einem Sulfonat, vorzugsweise einem Methansulfonat (Mesylat) wie Trifluormethansulfonat (Triflat) oder einem Benzolsulfonat wie p-Toluolsulfonat (Tosylat) und p-Brombenzolsulfonat (Brosylat), oder mit einer Diazoverbindung, z.B. Diazomethan.
   Als Basen eignen sich anorganische Basen, z.B. Carbonate wie Kaliumcarbonat und Natriumcarbonat, Hydrogencarbonate wie Kalium- und Natriumhydrogencarbonat, Alkalimetallhydride wie Natriumhydrid und Kaliumhydrid, sowie organische Basen, z.B. Amine wie Triethylamin, Pyridin und N,N-Diethylanilin, oder Alkalialkoholate wie Natriummethanolat, Natriumethanolat und Kalium-tert.-butanolat.
   Vorzugsweise verwendet man die 0,5- und 2-fache molare Menge sowohl an Base als auch an Alkylierungsmittel, bezogen auf die Menge an II (R⁵' = H) oder IV.
   Im allgemeinen empfiehlt sich eine Reaktionstemperatur von (-78°C) bis zur Siedetemperatur des Reaktionsgemisches, insbesondere von (- 60) bis 60°C.
   Üblicherweise entstehen bei der Alkylierung der Verbindungen der Formel II (mit R⁵ = Wasserstoff) oder IV neben den substituierten Zimtoxim-Derivaten I, bei denen Y für Sauerstoff oder Schwefel steht, auch die entsprechenden, Amid-Stickstoff substituierten Zimthydroxamid-Derivate (II). Das Verhältnis, in dem die beiden Produkte entstehen, hängt von der Reaktionstemperatur, vom Alkylierungsmittel, der verwendeten Base und auch von der jeweiligen Ausgangsverbindung II (mit R⁵ = Wasserstoff) oder IV ab. Nach den bisherigen Erkenntnissen entsteht meistens die Verbindung I im Überschuß. Sie kann normalerweise von den Nebenprodukten auf an sich bekannte Weise getrennt werden, z.B. durch Kristallisation oder Chromatographie.
b) Alkylierung eines substituierten Zimtoxim-Derivates I mit R⁶= Wasserstoff in Gegenwart einer Base: 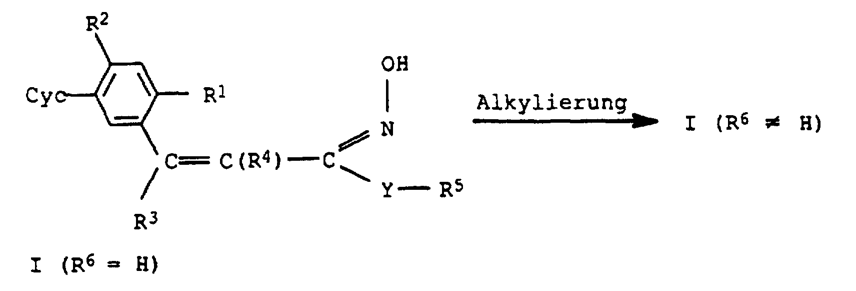 Bezüglich der Reaktionsbedingungen sei auf die Ausführungen unter Methode a) verwiesen.
c) Acylierung oder Sulfonylierung eines Zimthydroxamid-Derivats der Formel II mit R^{5'} = Wasserstoff oder der Formel IV: 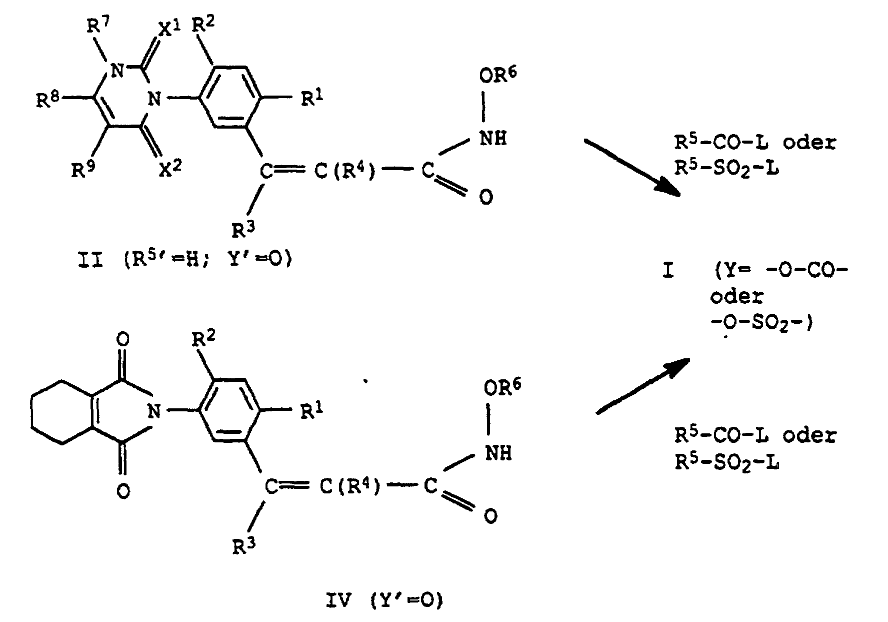
   L steht für eine übliche Abgangsgruppe wie Halogen, Alkylcarbonyloxy, Halogenalkylcarbonyloxy oder Imidazolyl.
   Normalerweise nimmt man die Umsetzung in einem inerten Lösungs- oder Verdünnungsmittel vor, wobei z.B. die für die Alkylierung unter a) genannten Solventien oder deren Mischungen in Betracht kommen.
   Das Acylierungsmittel kann auch "in situ" aus der entsprechenden Carbonsäure hergestellt werden, wobei dann bevorzugt . in Gegenwart eines üblichen Kondensationshilfsmittels gearbeitet wird. Geeignete Kondensationshilfsmittel sind z.B. Oxalylchlorid, Carbonyldiimidazol, Carbodiimide wie Dicyclohexylcarbodiimid, Halogenierungsmittel wie Thionylchlorid, Phoshoroxychlorid, Phosgen, Phosphortrichlorid und Phoshorpentachlorid, oder Chlorameisensäuremethylester oder -ethylester.
   Zweckmäßigerweise verwendet man etwa stöchiometrische Mengen an Acylierungsmittel und Zimthydroxamid-Derivat II (mit R^{5'} = Wasserstoff) oder IV, oder z.B. zur Optimierung des Umsatzes von II oder IV, einen Überschuß an Acylierungsmittel bis etwa 10 mol-%.
   In Abhängigkeit von den Ausgangsstoffen kann es vorteilhaft sein, in Gegenwart einer Base zu arbeiten. Als Base kommen hierfür anorganischen Basen, z.B. Carbonate wie Natrium- und Kaliumcarbonat, Alkalimetallhydrogencarbonate wie Natrium- und Kaliumhydrogencarbonat, Alkalimetallhydride wie Natrium- und Kaliumhydrid, sowie organische Basen, z.B. Amine wie Triethylamin, Pyridin und N,N-Diethylanilin, oder Alkalimetallalkoholate wie Natriummethanolat, Natriumethanolat und Kalium-tert.-butanolat in Betracht.
   Bereits eine katalytische Menge an Base von z.B. 0,01 mol-Äquivalenten, bezogen auf II oder IV, kann den Reaktionsverlauf positiv beeinflussen. Andererseits bringt eine Basenmenge über 200 mol-% normalerweise keine zusätzliohen Vorteile.
   Die Reaktion ist im allgemeinen bei einer Temperatur von (-20)°C bis zur Siedetemperatur des Reaktionsgemisches ausführbar. Vorzugsweise arbeitet man bei 0 bis 80°C.
d) Umsetzung eines Hydroximinohalogenids der Formel Ia mit einem Alkohol- oder Mercaptanderivat: 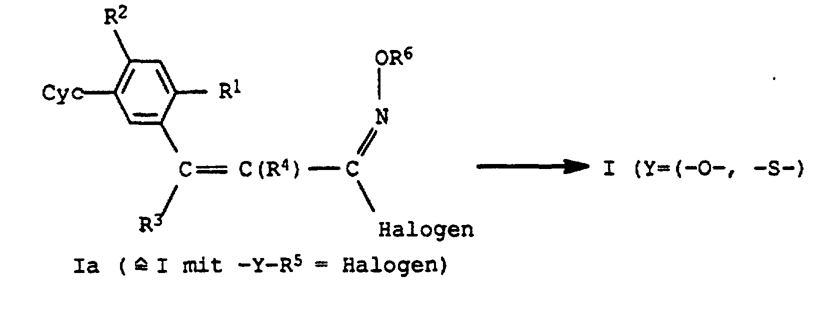
   Vorteilhaft verwendet man als Alkohol- oder Mercaptanderivate die Alkohole R₅-OH oder Mercaptane R₅-SH sowie deren Salze, insbesondere die der Alkali- oder Erdalkalimetalle.
   Geeignete Lösungs- oder Verdünnungsmittel sind z.B. aliphatische oder cyclische Ether wie Diethylether und Tetrahydrofuran, aliphatische Ketone wie Aceton, Kohlenwasserstoffe wie n-Pentan, Cyclohexan und Petrolether, aromatische Kohlenwasserstoffe wie Benzol und Toluol, halogenierte aliphatische oder aromatische Kohlenwasserstoffe wie Dichlormethan und Chlorbenzol, Ester wie Ethylacetat, Amide wie Dimethylformamid und N-Methyl-pyrrolidon, Sulfoxide wie Dimethylsulfoxid, sowie Mischungen dieser Solventien. Auch die Alkohol- und Mercaptanderivate selbst kommen als Lösungs- oder Verdünnungsmittel in Betracht.
   Das Mengenverhältnis von Ia zu Alkohol- oder Mercaptanderivat ist nicht kritisch. Üblicherweise werden etwa äquimolare Mengen eingesetzt. Es kann aber auch zweckmäßig sein, das Alkohol- oder Mercaptanderivat in einem Überschuß einzusetzen, so daß es gleichzeitig als Lösungs- oder Verdünnungsmittel dient.
   Im allgemeinen empfiehlt sich eine Reaktionstemperatur von (-78)°C bis zur Rückflußtemperatur des verwendeten Lösungsmittels, insbesondere von 0 bis 80°C.
   Bei der Umsetzung von Ia mit einem Alkohol R⁵-OH oder Mercaptan R⁵-SH arbeitet man besonders vorteilhaft in Gegenwart einer Base, wobei sowohl anorganische Basen, z.B. Carbonate, Hydrogencarbonate oder Alkalimetallhydride, als auch organische Basen, z.B. Amine wie Triethylamin, Pyridin und N,N-Dimethylanilin, oder Alkalimetallalkoholate, in Betracht kommen. Zweckmäßigerweise verwendet man ein Alkoholat des Alkohols R⁵-OH.
   Die Base kann in katalytischer, unterstöchiometrischer, stöchiometrischer Menge oder im Überschuß, bis zur 5-fachen molaren Menge, bezogen auf I, eingesetzt werden.
e) Halogenierung von Verbindungen der Formel II mit Y' = Sauerstoff und R₅' = Wasserstoff oder der Formel IV mit Y' = Sauerstoff: 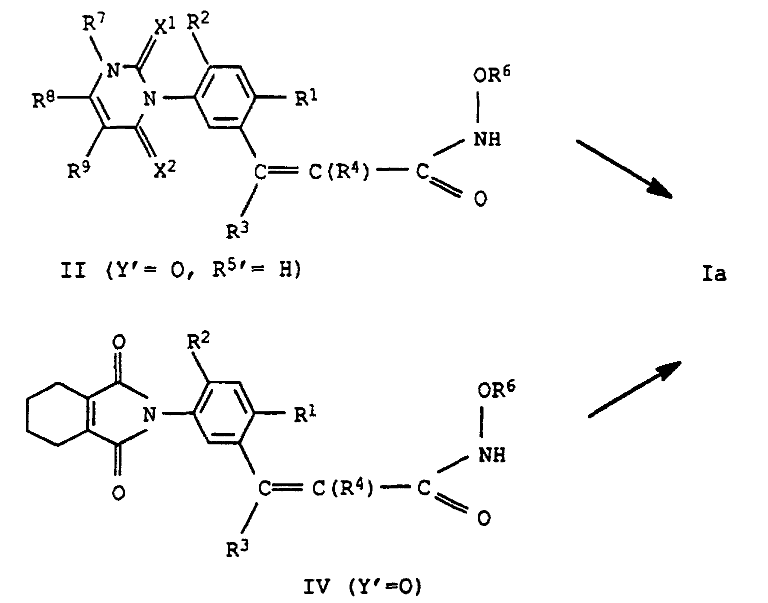
   Üblicherweise arbeitet man in einem inerten Lösungs- oder Verdünnungsmittel, wobei insbesondere aprotische organische Flüssigkeiten, beispielsweise aliphatische oder aromatische Kohlenwasserstoffe wie n-Hexan, Benzol, Toluol und o-, m-, p-Xylol, halogenierte aliphatische Kohlenwasserstoffe wie Methylenchlorid, Chloroform und 1,2-Dichlorethan, halogenierte aromatische Kohlenwasserstoffe wie Chlorbenzol, tertiäre Amine wie N,N-Dimethylanilin, oder Nitrile wie Acetonitril in Betracht kommen.
   Als Halogenierungsmittel eignen sich vor allem Thionylchlorid, Phosphorpentachlorid, Phosphoroxychlorid, Phosphorpentabromid oder Phosphoroxybromid. Besonders vorteilhaft kann auch die Verwendung eines Gemisches aus Phosphorpentachlorid und Phosphoroxychlorid oder aus Phosphorpentabromid und Phosphoroxybromid sein, wobei dann ohne Verdünnungsmittel in einem Überschuß an Phosphoroxychlorid oder Phosphoroxybromid gearbeitet werden kann.
   Bei Verwendung von Thionylchlorid als Halogenierungsmittel empfiehlt es sich, eine katalytische Menge an Dimethylformamid zuzusetzen.
   Besonders bewährt hat sich ein Gemisch aus einem Tetrahalogenmethan wie Tetrachlor- und Tetrabromkohlenstoff, und einem unsubstituierten oder substituierten Triphenylphosphan, z.B. Triphenylphosphan oder Tri-(o-tolyl)-phosphan.
   Für eine vollständige Umsetzung benötigt man mindestens äquimolare Mengen an Halogenierungsmittel und Ausgangsverbindung II (Y' = O, R⁵' = H) oder IV (Y' = O). Im allgemeinen wirkt sich ein Überschuß an Halogenierungsmittel, bis etwa zur 8-fachen molaren Menge, bezogen auf II oder IV, günstig auf den Reaktionsverlauf aus.
   Die Reaktionstemperatur liegt im allgemeinen bei 0°C bis zur Rückflußtemperatur des Reaktionsgemisches, vorzugsweise bei 20 bis 120°C.
f) Überführung eines Zimtnitrils V in eine Verbindung der Formel I, wobei Y Sauerstoff oder Schwefel bedeutet: 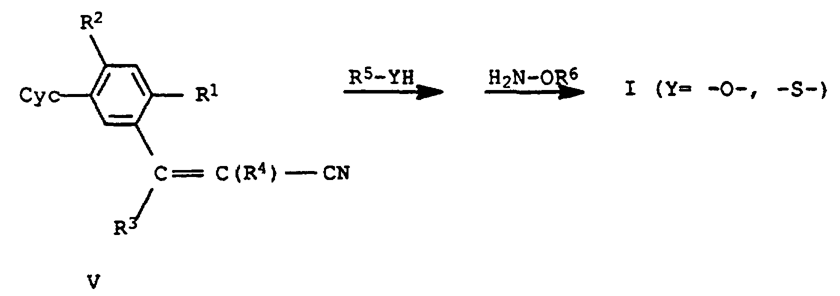
   Die Reaktion erfolgt üblicherweise in zwei Stufen, indem man das Zimtnitril V zuerst mit einem Alkohol oder Mercaptan R⁵-YH umsetzt und den hierbei erhaltenen Imidoester oder Thioimidoester VI, gewünschtenfalls ohne Isolierung aus der Reaktionsmischung, mit einem Hydroxylamin H₂N-OR⁶ zur Reaktion bringt.
   Die Umsetzung von V mit R⁵-YH kann in einem inerten Lösungs- oder Verdünnungsmittel oder lösungsmittelfrei in einem Überschuß des Alkohols oder Mercaptans durchgeführt werden. Häufig ist ein saurer oder "Lewis"-saurer Katalysator förderlich, vorzugsweise in etwa katalytischer Menge oder in einer Menge bis etwa 200 mol-%, bezogen auf die Menge an V.
   Als inerte Lösungs- oder Verdünnungsmittel eignen sich besonders organische Lösungsmittel, z.B. aliphatische oder cyclische Ether wie Diethylether, Tetrahydrofuran und Dimethoxyethan, aliphatische, cyclische oder aromatische Kohlenwasserstoffe wie n-Pentan, Petrolether, Cyclohexan, Toluol und die Xylole, Amide wie Dimethylformamid und N-Methylpyrrolidon, halogenierte Kohlenwasserstoffe wie Dichlormethan, Chlorbenzol und 1,2-Dichlormethan, oder Mischungen der genannten Solventien.
   Als saure Katalysatoren eignen sich anorganische, bevorzugt wasserfreie Säuren, z. B. Chlorwasserstoff, Bromwasserstoff, Salpetersäure, Schwefelsäure, auch Oleum, oder Perchlorsäure, sowie organische Säuren wie Essigsäure, Propionsäure, p-Toluolsulfonsäure und Trifluoressigsäure. Beispiele für "Lewis"-saure Katalysatoren sind Titantetrachlorid, Zinn(II)-Chlorid, Eisen(III)-Chlorid, Aluminiumtrichlorid, Ethylaluminiumdichlorid, Titantetraisopropylat und Bortrifluoroetherat.
   Die Menge an Alkohol oder Mercaptan ist nicht kritisch. Normalerweise sind 1 bis 10 mol Alkohol oder Mercaptan pro mol V für eine optimale Umsetzung von V ausreichend. Arbeitet man lösungsmittelfrei in dem betreffenden Alkohol, so kann dieser auch in einem größeren Überschuß vorliegen.
   Sofern der Imidoester oder Thioimidoester VI in der ersten Stufe als Salz anfällt, empfiehlt es sich, die Neutralverbindung freizusetzen, bevor die Umsetzung mit dem Hydroxylamin H₂N-OR⁶ vorgenommen wird.
   Hydroxylamine, die in Form ihrer Salze, insbesondere als Hydrochloride, Hydrobromide oder Sulfate, erhältlich sind oder bei der Herstellung als Salze anfallen, können vor ihrer Umsetzung durch Zugabe einer geeigneten Base freigesetzt werden, wobei als Basen insbesondere die bei Methode a) genannten geeignet sind.
   Die Umsetzung des erhaltenen Imidoesters oder Thioimidoesters VI mit H₂N-OR⁶ erfolgt im allgemeinen in einem inerten Lösungs- oder Verdünnungsmittel. Hierfür kommen neben den vorstehend genannten Solventien zusätzlich Alkohole wie Methanol, Ethanol und Isopropanol, Nitrile wie Acetonitril, Amine wie Triethylamin, Pyridin und N,N-Dimethylanilin, oder auch Wasser in Betracht.
   (Thio)imidoester VI und Hydroxylamin werden zweckmäßigerweise in etwa äquimolaren Mengen miteinander umgesetzt. Um den (Thio)imidoester VI möglichst vollständig umzusetzen kann es aber empfehlenswert sein, das Hydroxylamin H₂N-OR⁶ in einem Überschuß, bis etwa 10 mol-%, einzusetzen.
   Die Reaktionstemperatur liegt für beide Stufen im allgemeinen bei (-20) bis 120°C, insbesondere bei 0°C bis zur Siedetemperatur des Reaktionsgemisches.
g) Oximierung eines Zimtaldehyds bzw.-Ketons der Formel VII: 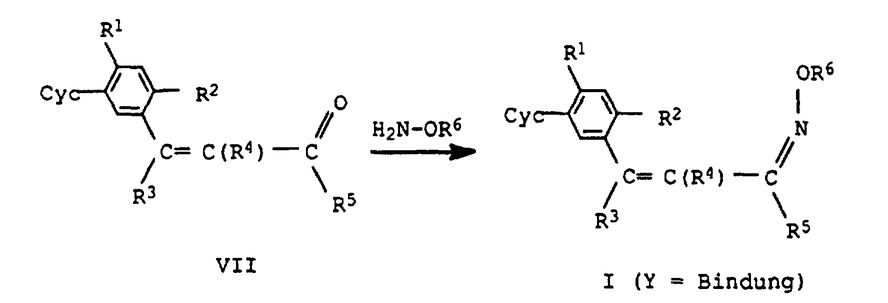
   Die Umsetzung von VII mit einem Hydroxylamin H₂N-OR⁶ erfolgt normalerweise in einem inerten organischen Lösungs- oder Verdünnungsmittel, z.B. in einem aromatischen Kohlenwasserstoff wie Toluol und die Xylole, in einem chlorierten Kohlenwasserstoff wie Dichlormethan, Choroform und Chlorbenzol, in einem Ether wie Diethylether, 1,2-Dimethoxyether und Tetrahydrofuran, in einem Alkohol wie Methanol und Ethanol, in Wasser oder in einem Gemisch der genannten Lösungsmittel.
   Sofern die Hydroxylamine H₂N-OR⁶ als Salze, z.B. als Hydrochloride oder Oxalate vorliegen, ist ihre Freisetzung mittels Base wie vorzugsweise Natriumcarbonat, Kaliumcarbonat, Natriumhydrogencarbonat, Triethylamin und Pyridin, empfehlenswert.
   Die Menge an Hydroxylamin beträgt vorzugsweise 80 bis 800 mol-%, insbesondere 100 bis 300 mol-%, bezogen auf die Menge an VII.
   Das entstehende Reaktionswasser kann gewünschtenfalls destillativ oder mit Hilfe eines Wasserabscheiders aus dem Reaktionsgemisch entfernt werden.
   Üblicherweise liegt die Reaktionstemperatur bei (-30) bis 150°C, bevorzugt bei 0 bis 130°C.
h) Überführung eines Zimtoxims der Formel VIII 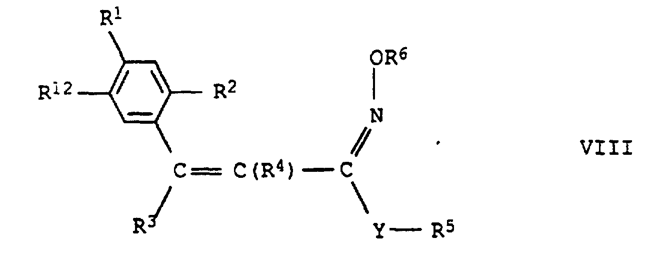 wobei R¹² für Nitro, Amino, Isocyanato, Isothiocyanato, (C₁-C₆-Alkyl)carbamato oder Phenylcarbamato steht,
   nach einem in der WO 93/06090 beschriebenen Verfahren in die substituierten Zimtoxim-Derivate I.

Die Verbindungen der Formel VIII sind neu. Sie sind ihrerseits nach einem der vorstehend zur Herstellung von Verbindungen I beschriebenen Verfahren erhältlich. Weitere Herstellungsmethoden zur Bereitstellung der Verbindungen VIII können ferner der WO 93/06090 entnommen werden.

Die Verbindungen der Formel V sind bekannt oder lassen sich auf an sich bekannte Weise herstellen (vgl. z. B. WO 93/06090).

Die Zimthydroxamid-Derivate der Formeln II und IV (mit Y' - Sauerstoff) sind z.B. aus Zimtsäuren der Formeln IX und X zugänglich:

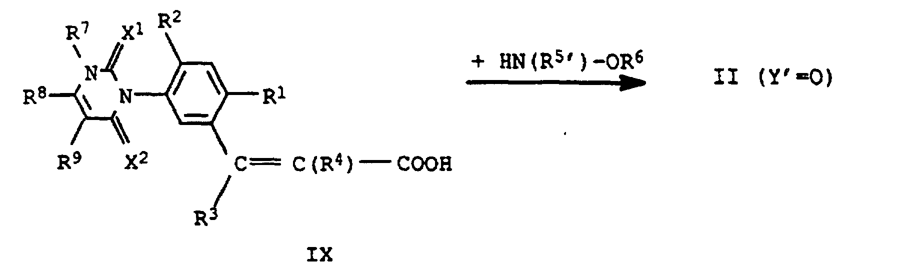

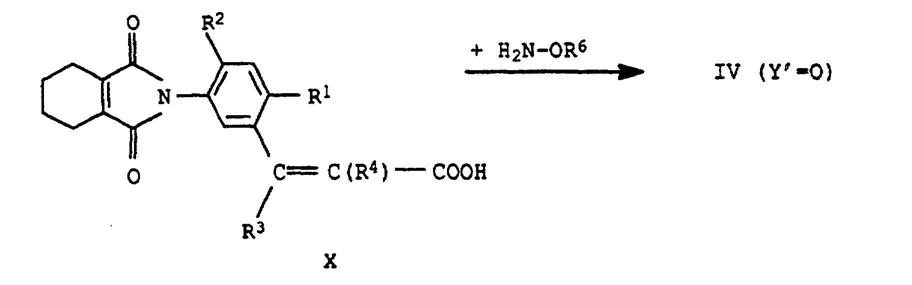

Die Reaktion wird Üblicherweise in einem inerten Lösungs- oder Verdünnungsmittel in Gegenwart eines Kondensationshilfsmittels oder ohne Lösungsmittel in einem Überschuß des Kondensationshilfsmittels vorgenommen.

Als Lösungs- oder Verdünnungsmittel eignen sich insbesondere organische Lösungsmittel, z.B. aliphatische oder cyclische Ether, wie Diethylether, Tetrahydrofuran und Dimethoxyethan, aliphatische, cyclische oder aromatische Kohlenwasserstoffe wie n-Pentan, Petrolether, Cyclohexan, Toluol und die Xylole, Alkohole wie Methanol, Ethanol und i-Propanol, Amide wie Dimethylformamid und N-Methylpyrrolidon, Nitrile wie Acetonitril, Amine wie Triethylamin, Pyridin und N,N-Dimethylanilin, halogenierten Kohlenwasserstoffe wie Dichlormethan, Chlorbenzol und 1,2-Dichlormethan, oder Wasser. Auch Mischungen aus den genannten Solventien sind geeignet.

Als Kondensationshilfsmittel kommen z.B. Oxalylchlorid, Carbonyldiimidazol, Carbodiimide wie Dicyclohexylcarbodiimid, Halogenierungsmittel wie Thionylchlorid, Phoshoroxychlorid, Phosgen, Phosphortrichlorid und Phoshorpentachlorid, oder Chlorameisensäuremethylester oder -ethylester in Betracht.

Bevorzugt ist die Verwendung eines Halogenierungsmittels, wobei zuerst "in situ" ein Säurehalogenid entsteht, das dann mit dem Hydroxylamin HN(R⁵')-OR⁶ oder H₂N-OR⁶ zum Produkt II oder IV weiterreagiert.

Es besteht aber auch die Möglichkeit, das Säurehalogenid in einem separaten Verfahrensschritt gezielt herzustellen und, gewünschtenfalls in gereinigter Form, danach mit dem Hydroxylamin HN(R⁵')-OR⁶ oder H₂N-OR⁶ umzusetzen.

Hydroxylamine, die in Form ihrer Salze, insbesondere als Hydrochloride, Hydrobromide oder Sulfate, erhältlich sind oder bei der Herstellung als Salze anfallen, können vor ihrer Umsetzung mit IX oder X, gewünschtenfalls auch in der Reaktionsmischung mit dem Kondensationshilfsmittel und IX oder X, durch Zugabe einer geeigneten Base freigesetzt werden.

Als Basen eignen sich hierfür insbesondere die bei Methode a) genannten.

Die Mengen an Kondensationshilfsmittel, IX oder X und Hydroxylamin HN(R⁵')-OR⁶ oder H₂N-OR⁶ sind nicht kritisch. Zweckmäßigerweise verwendet man etwa äquimolare Mengen der Ausgangsstoffe. Das Kondensationshilfsmittel kann gewünschtenfalls auch im Überschuß eingesetzt werden, wobei dann sogar ohne inertes Lösungsmittel gearbeitet werden kann.

Alle vorstehend beschriebenen Verfahren werden zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Sowohl die substituierten Zimtoxim-Derivate der Formel I als auch die substituierten Zimthydroxamid-Derivate der Formel II können bei der Herstellung als Isomerengemische anfallen, die gewünschtenfalls nach den hierfür üblichen Methoden, z.B. mittels Kristallisation oder Chromatographie an einem optisch aktiven Adsorbat, in die reinen Isomeren getrennt werden können. Reine optisch aktive Isomere lassen sich beispielsweise auch aus entsprechenden optisch aktiven Ausgangsmaterialien herstellen.

Substituierten Zimtoxim-Derivate I und Zimthydroxamid-Derivate II mit C-H aciden Substituenten lassen sich auf an sich bekannte Weise in ihre Alkalimetallsalze überführen.

Salze von I oder II, deren Metallion kein Alkalimetallion ist, können üblicherweise durch Umsalzen des entsprechenden Alkalimetallsalzes in wäßriger Lösung hergestellt werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Die Verbindungen I und II sowie deren landwirtschaftlich brauchbaren Salze eignen sich, sowohl als Isomerengemische als auch in Form der reinen Isomeren, als Herbizide. Sie können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I und II bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa , Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus lassen sich die Verbindungen I und II auch in Kulturen, die durch Züchtung und/oder mittels gentechnischer Methoden gegen die Wirkung von I bzw. II oder anderen Herbiziden weitgehend resistent gemacht wurden, einsetzen.

Des weiteren eignen sich die substituierten Zimtoxim-Derivate I und Zimthydroxamid-Derivate II auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I und II bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I und II in den anwendungsfertigen Zubereitungen können in weiten Bereichen variiert werden, etwa von 0,01 bis 95 Gew.%, vorzugsweise 0,5 bis 90 Gew.%. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 1.01 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. I.02 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III.20 Gewichtsteile des Wirkstoffs Nr. I.05 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. I.08 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.
V. 3 Gewichtsteile des Wirkstoffs Nr. 1.09 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. 1.13 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Wirkstoffe I und II bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

Zur Verbreiterung des Wirkungsspektrums und zur Erzielung synergistischer Effekte können die substituierten Zimtoxim-Derivate I und die substituierten Zimthydroxamid-Derivate II mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I oder II allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele:

### Beispiel 1

### 3-[4-Chlor-3-(2-chlor-2-(ethoxiaminocarbonyl)-ethenyl)-phenyl]-2,4-dioxo-1-methyl-6-trifluormethyl-1,2,3,4-tetrahydropyrimidin (Verbindung Nr. II.02)

Zu einer Lösung von 3-[3-(2-Carboxy-2-chlor-ethenyl)-phenyl]-2,4-dioxo-1-methyl-6-trifluormethyl-1,2,3,4-tetrahydropyrimidin (2,1 g) in 80 ml Tetrahydrofuran wurde Carbonyldiimidazol (0,9 g) gegeben, wonach man die Mischung eine Stunde bei 25°C rührte. Anschließend wurde Ethoxyamin (0,34 g), gelöst in 20 ml Tetrahydrofuran, zugetropft. Nach fünfstündigem Rühren entfernte man das Lösungsmittel. Der Rückstand wurde in 150 ml Dichlormethan aufgenommen. Die organische Phase wurde je zweimal mit 30 ml Wasser, 30 ml 10 gew.-%iger Natriumhydrogencarbonatlösung und nochmals 30 ml Wasser gewaschen, schließlich über Natriumsulfat getrocknet und eingeengt. Nach Kristallisation aus Petrolether erhielt man 2,0 g des Produkts. Smp.: 86 - 90°C.

### Beispiel 2

### 3-[4-Chlor-3-(2-chlor-2-methoxiaminocarbonylethenyl)-phenyl]-2,4-dioxo-1-methyl-6-trifluormethyl-1,2,3,4-tetrahydropyrimidin (Verbindung II.01)

Zu einer Lösung von 3-[3-(2-carboxy-2-chlor-ethenyl)-phenyl]-2,4-dioxo-1-methyl-6-trifluormethyl-1,2,3,4-tetrahydropyrimidin (2,1 g) in 80 ml Tetrahydrofuran wurde Carbonyldiimidazol (0,9 g) gegeben, wonach man die Mischung eine Stunde bei 25°C rührte. Anschließend wurde Methoxiamin-Hydrochlorid (1,67 g als 30 gew.-%ige wäßrige Lösung) und Kaliumcarbonat (0,83 g), gelöst in 20 ml Tetrahydrofuran, zugetropft. Nach 5 Stunden Rühren destillierte man das Lösungsmittel ab, wonach der Rückstand in 150 ml Dichlormethan aufgenommen wurde. Die organische Phase wurde zweimal mit je 30 ml Wasser, zweimal mit je 30 ml 10 gew.%iger wäßriger Natriumhydrogencarbonat-Lösung und nochmals mit 30 ml Wasser gewaschen, dann über Natriumsulfat getrocknet und eingeengt. Nach Kristallisation mit Petrolether erhielt man 1,5 g des Produkts; Smp.: 136 -141°C.

### Beispiel 3

### 3-[4-Chlor-3-(2-chlor-3-methoximino-3-methoxy-propenyl)-phenyl]-2,4-dioxo-1-methyl-6-trifluormethyl-1,2,3,4-tetrahydropyrimidin (Verbindung I.01) und 3-[4-Chlor-3-(2-chlor-3-[methoxy-methylamino- carbonyl]-ethenyl)-phenyl]-2,4-dioxo-1-methyl-6-trifluormethyl-1,2,3,4-tetrahydropyrimidin (Verbindung II.03)

Zu einer Lösung von 3-[4-Chlor-3-(2-chlor-2-methoxyaminocarbonyl-ethenyl)-phenyl]-2,4-dioxo-1-methyl-6-trifluormethyl-1,2,3,4-tetrahydropyrimidin (2,2 g) in 80 ml Aceton wurden erst Kaliumcarbonat (0,76 g) und dann in 20 ml Aceton gelöstes Dimethylsulfat (0,63 g) gegeben. Nach 17 Std. Rühren wurde nochmals Dimethylsulfat (0,13 g) zugegeben, wonach man erneut 17 Std. rührte und anschließend das Lösungsmittel abdestillierte. Der Rückstand wurde in 100 ml Dichlormethan aufgenommen. Die organische Phase wurde dreimal mit je 30 ml Wasser gewaschen, über Natriumsulfat getrocknet und eingeengt. Nach Reinigung des Rohprodukts mittels Chromatographie und Kristallisation erhielt man 1,0 g 3-[4-Chlor-3-(2-chlor-3-methoximino-3-methoxy-propenyl)-phenyl]-2,4-dioxo-1-methyl-6-trifluormethyl-1,2,3,4-tetrahydropyrimidin (Verbindung I.01; Smp. 152-157°C) und 0,4 g 3-[4-Chlor-3-(2-chlor-3-[methoxy-methylamino-carbonyl]-ethenyl)-phenyl]-2,4-dioxo-1-methyl-6-trifluormethyl-1,2,3,4-tetrahydropyrimidin (Verbindung II.03; Smp.: 121 -123°C).

### Beispiel 4

### 3-[4-Chlor-3-(2-chlor-3-methoximino-3-methoxyethoxy-propenyl)-phenyl]-2,4-dioxo-1-methyl-6-trifluormethyl-1,2,3,4-tetrahydropyrimidin (Verbindung I.03) und 3-[4-Chlor-3-(2-methoximino-3-methoxyethoxy)-ethinyl-phenyl]-2,4-dioxo-1-methyl-6-trifluormethyl-1,2,3,4-tetrahydropyrimidin (Verbindung I.18)

Zu einer Lösung von 3-[4-Chlor-3-(2-chlor-2-methoxyaminocarbonyl-ethenyl)-phenyl]-2,4-dioxo-1-methyl-6-trifluormethyl-1,2,3,4-tetrahydropyrimidin (4,4 g) in einem Gemisch aus 10 ml Aceton und 5 ml 1,2-Dimethyltetrahydro-2(1H)-pyrimidinon wurden Kaliumcarbonat (1,4 g) und dann in 5 ml Aceton gelöstes Methoxyethoxytosylat (2,3 g) gegeben. Nach 2 Tagen Rühren bei 25°C gab man N,N-Dimethylaminopyridin (0,2 g) in das Reaktionsgemisch. Nach 10 Std. Rühren bei Rückflußtemperatur wurde das Lösungsmittel abdestilliert, wonach man den Rückstand in 150 ml Dichlormethan aufnahm. Die Dichlormethanphase wurde dreimal mit je 50 ml Wasser gewaschen, über Natriumsulfat getrocknet und eingeengt. Nach zweimaliger Flash-Chromatographie erhielt man 0,4 g 3-[4-Chlor-3-(2-chlor-3-methoximino-3-methoxyethoxy-propenyl)phenyl]-2,4-dioxo-1-methyl-6-trifluormethyl-1,2,3,4- tetrahydropyrimidin, (Verbindung I.03: Smp.: 73-74°C) und 0,12 g 3-[4-Chlor-3-(2-methoximino-3-methoxyethoxy-ethinyl-phenyl]-2,4-dioxo-1-methyl-6- trifluormethyl-1,2,3,4-tetrahydropyrimidine (Verbindung I.18; Smp.: 127-129).

### Beispiel 5

### 3-[4-Chlor-3-(2-chlor-3-[3-propen-oximino]-butenyl)-phenyl]-2,4-dioxo-1-methyl-6-trifluormethyl-1,2,3,4-tetrahydropyrimidin (Verbindung I.06)

Zu einer Lösung von 3-[4-Chlor-3-(2-chlor-3-oxo-butenyl)-phenyl]-2,4-dioxo-1-methyl-6-trifluormethyl-1,2,3,4-tetrahydropyrimidin (2,04 g) in 100 ml Toluol wurden Natriumcarbonat (0,69 g) und O-3-Propenyl-hydroxylaminhydrochlorid (0,66 g) gegeben. Nach 5 Std. Rühren bei 25°C erhitzte man noch 20 Std. auf Rückflußtemperatur, wobei insgesamt nochmals 0,53 g Natriumcarbonat und 0,55 g O-3-Propenyl-hydroxylaminhydrochlorid zugegeben wurden. Anschließend wurde die organischen Phase dreimal mit je 50 ml Wasser gewaschen, über Natriumsulfat getrocknet und eingeengt. Nach Kristallisation erhielt man 1,0 g des Produkts; Smp 72 - 74°C.

### Beispiel 6

### 3-[3-(3-Acetoxy-2-chlor-3-methoximino-propenyl)-4-chlor-phenyl]-2,4-dioxo-1-methyl-6-trifluormethyl-1,2,3,4-tetrahydropyrimidin (Verbindung I.12) und 3-[3-(2-Acetylmethoxy-aminocarbonyl-2-chlor-ethenyl)-phenyl]-2,4-dioxo-1-methyl-6-trifluormethyl-1,2,3,4-tetrahydropyrimidin (Verbindung II.05).

Zu einer Lösung von 3-[4-Chlor-3-(2-chlor-3-methoxyaminocarbonylethenyl)-phenyl]-2,4-dioxo-1-methyl-6-trifluormethyl-1,2,3,4-tetrahydropyrimidin (2,2 g) und Triethylamin (0,77 ml) in 80 ml Dichlormethan wurde bei 25°C eine Lösung von Acetylchlorid (0,39 ml) in 20 ml Dichlormethan getropft. Nach 20 Stunden Rühren bei ca. 20°C wurde die organische Phase dreimal mit je 30 ml Wasser gewaschen, dann getrocknet und eingeengt. Die Reinigung des Rohprodukts erfolgte chromatographisch (Laufmittel: Dichlormethan/Ethylacetat = 9:1). Ausbeute: 1,1 g 3-[3-(3-Acetoxy-2-chlor-3-methoximino-propenyl)-4-chlor-phenyl]-2,4-dioxo-1-methyl-6-trifluormethyl-1,2,3,4-tetrahydropyrimidin (Smp.: 158-160°C) und 0,2 g 3-[3-(2-[Acetylmethoxyaminocarbonyl]-2-chlor-ethenyl)-phenyl]-2,4-dioxo-1-methyl-6-trifluormethyl-1,2,3,4-tetrahydropyrimidin (Smp.: 117-118°C).

### Beispiel 7

### 3-[3-(3-Brom-2-chlor-3-methoximino-propenyl)-4-chlor-phenyl]-2,4-dioxo-1-methyl-6-trifluormethyl-1,2,3,4-tetrahydropyrimidin (Verbindung I.13).

Zu einer Lösung von 3-[4-Chlor-(2-chlor-2-methoxyaminocarbonylethenyl)-phenyl]-2,4-dioxo-1-methyl-6-trifluormethyl-1,2,3,4-tetrahydropyrimidin (4,4 g) und Triphenylphosphin (3,9 g) in 100 ml Acetonitril wurde Tetrabrommethan (5,0 g) gegeben. Anschließend erhitzte man das Gemisch 35 Std. auf Rückflußtemperatur, währenddessen zweimal Triphenylphosphin (6,5 g) und Tetrabrommethan (8,3 g) zugegeben wurden. Nach Abkühlen der Reaktionsmischung entfernte man das Lösungsmittel. Der Rückstand wurde chromatographisch gereinigt (Laufmittel: Dichlormethan). Ausbeute: 3,0 g; Smp.: 128-130°C.

### Beispiel 8

### 3-[4-Chlor-3-(2-chlor-3-ethylthio-3-methoximino-propenyl)-phenyl]-2,4-dioxo-1-methyl-6-trifluormethyl-1,2,3,4-tetrahydropyrimidin (Verbindung I.19)

Zu einer Suspension von Natriumhydrid (0,18 g; 80 gew%ig in Weißöl) in 100 ml Tetrahydrofuran wurde unter Stickstoff eine Lösung von Ethylmercaptan (0,34 g) in 20 ml Tetrahydrofuran gegeben. Nach 30 Minuten Rühren versetzte man das Gemisch mit einer Lösung von 3-[3-(3-Brom-2-chlor-3-methoximino-propenyl)-4-chlorphenyl]-2,4-dioxo-1-methyl-6-trifluormethyl-1,2,3,4-tetrahydropyrimidin (2,5 g; hergestellt nach Beispiel 7) in 30 ml Tetrahydrofuran. Anschließend rührte man 20 Stunden, wonach nochmals Ethylmercaptan (0,34 g) und Natriumhydrid (0,34 g; 80 %ig in Weißöl) zugegeben wurden. Dann rührte man weitere 20 Stunden. Zur Aufarbeitung gab man die Reaktionsmischung in 150 ml Wasser. Das Produkt wurde mit Dichlormethan (zweimal je 100 ml) aus der wäßrigen Phase extrahiert. Die vereinigten organischen Phasen wurden dreimal mit je 50 ml Wasser gewaschen, über Natriumsulfat getrocknet und eingeengt. Die Reinigung des Rohprodukts erfolgte chromatographisch.

### Beispiel 9 (Verbindung I.28)

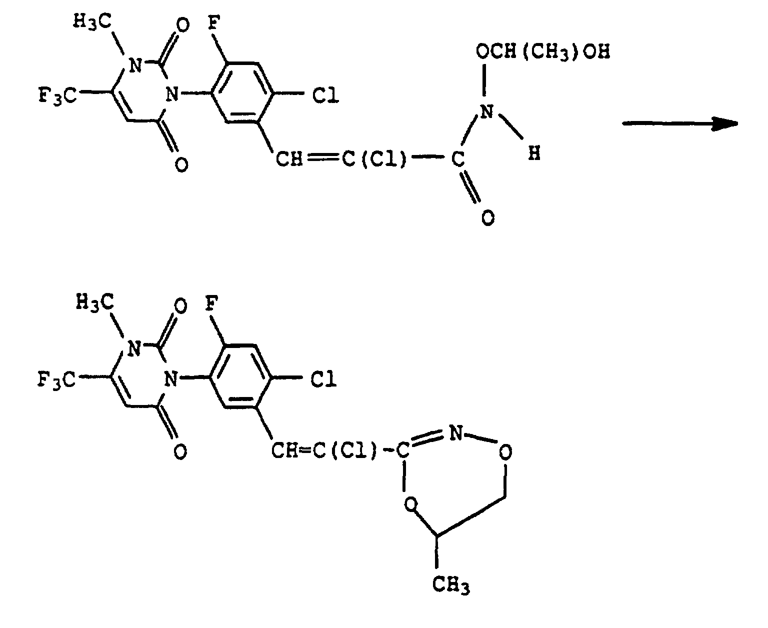

Zu einer Mischung aus 0,004 Mol 3-[4-Chlor-3-(2-Chlor-2-[(1-hydroxyethoxy)aminocarbonyl]-ethenyl)-2-fluor-phenyl]-2,4-dioxo-1-methyl-6-trifluormethyl-1,2,3,4-tetrahydropyrimidin, 0,0048 Mol Kaliumcarbonat und 80 ml Aceton wurden bei ca. 25°C langsam 0,0048 mol Dimethylsulfat in 20 ml Aceton getropft. Anschließend rührte man 12 Std. und engte dann ein. Der Rückstand wurde in 100 ml Methylenchlorid aufgenommen, wonach die Methylenchlorid-Phase 3mal mit je 30 ml Wasser gewaschen, dann über Natriumsulfat getrocknet und schließlich eingeengt wurde. Die Reinigung des Rohprodukts erfolgte chromatographisch an Kieselgel.

In den folgenden Tabellen 3 bis 5 sind die vorstehend genannten Verbindungen zusammen mit weiteren substituierten Zimtoxim-Derivaten I, die auf die gleiche Weise hergestellt wurden oder herstellbar sind, aufgeführt:

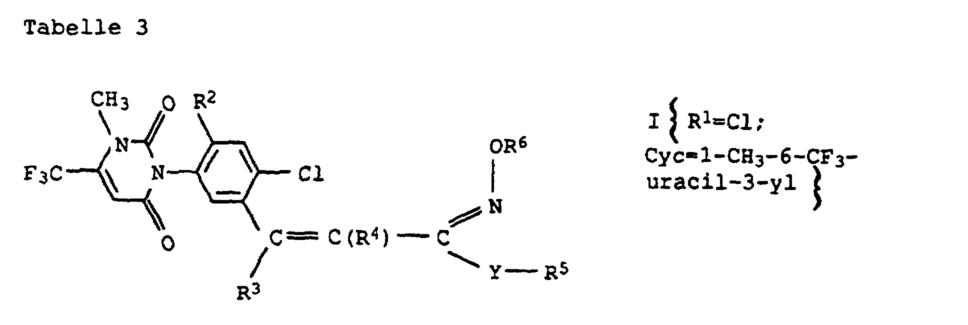

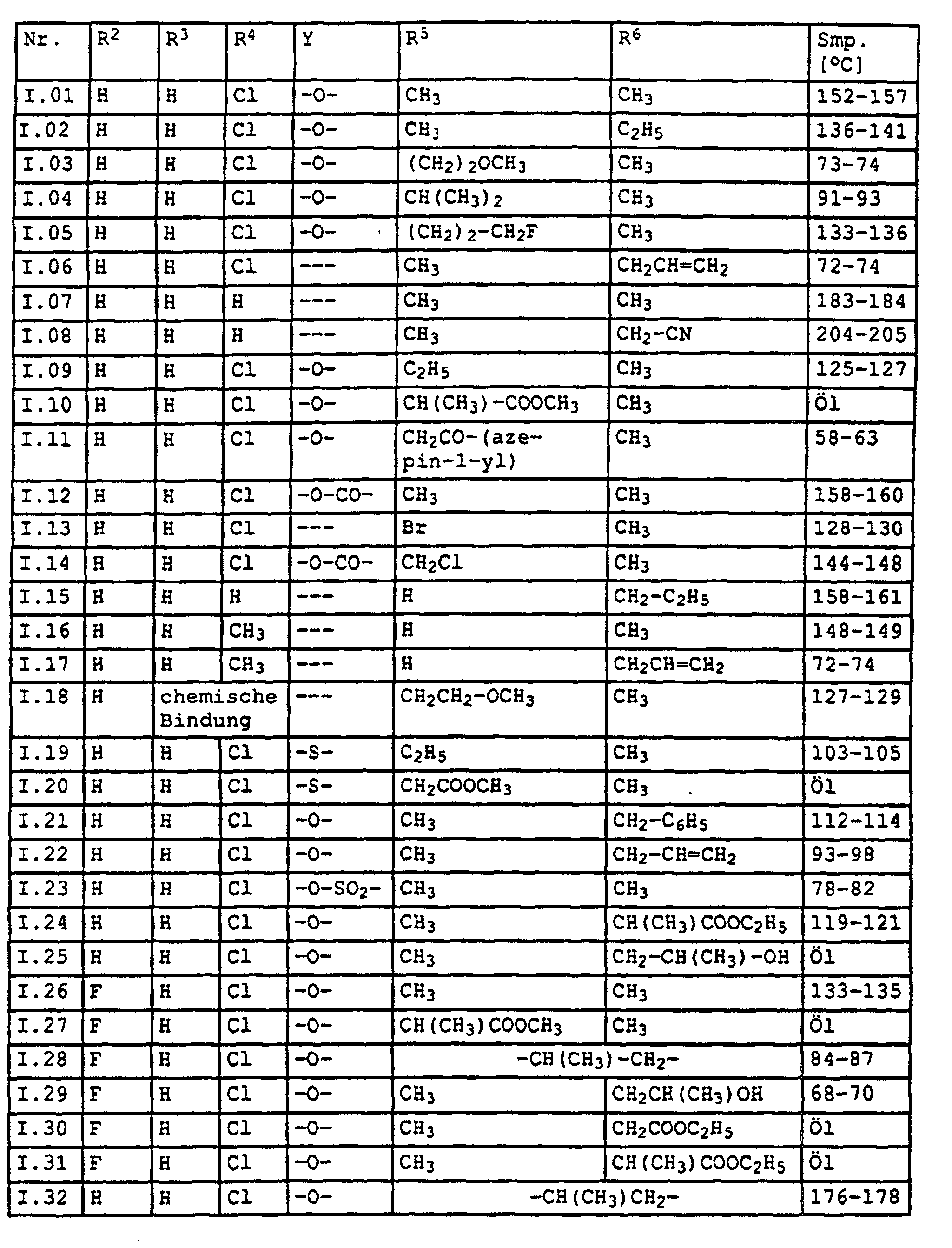

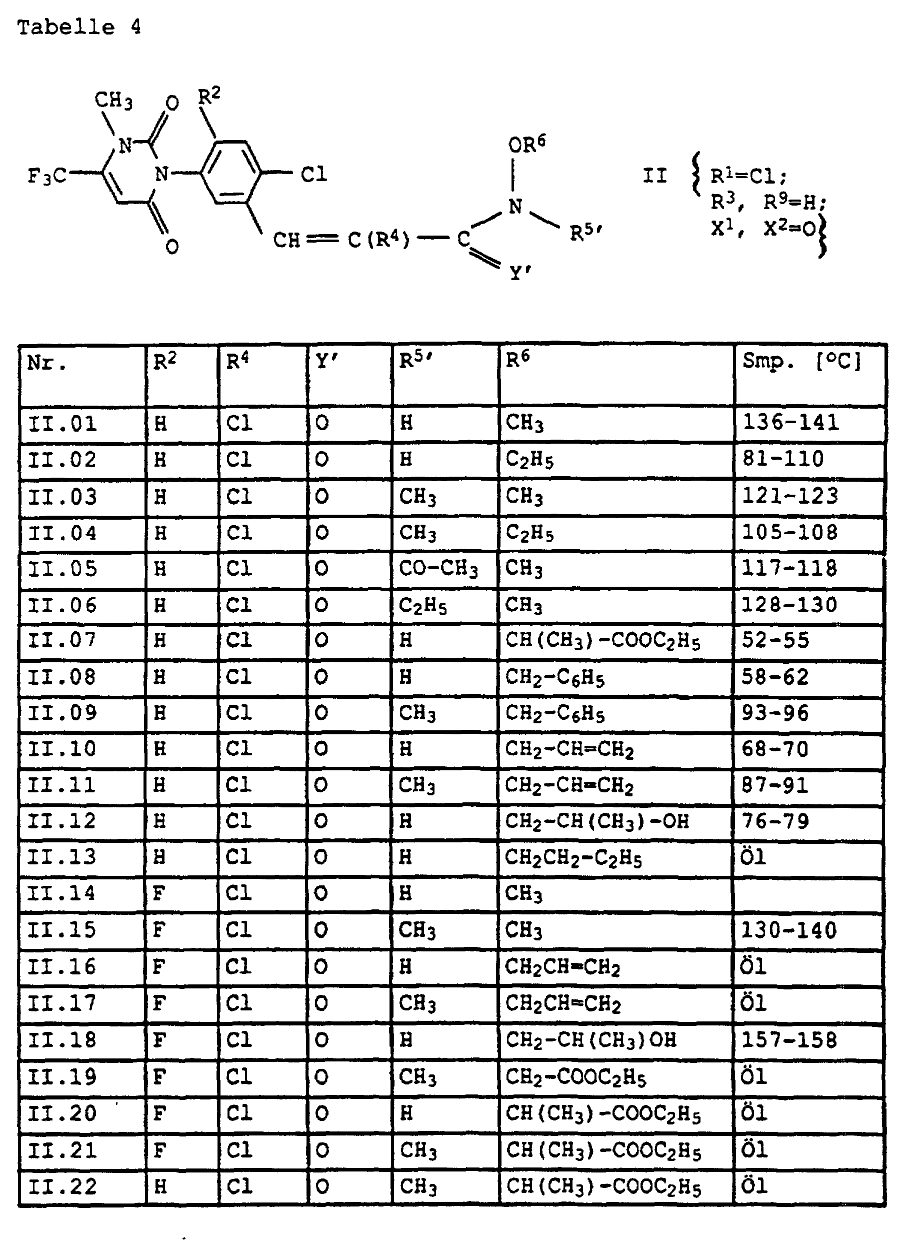

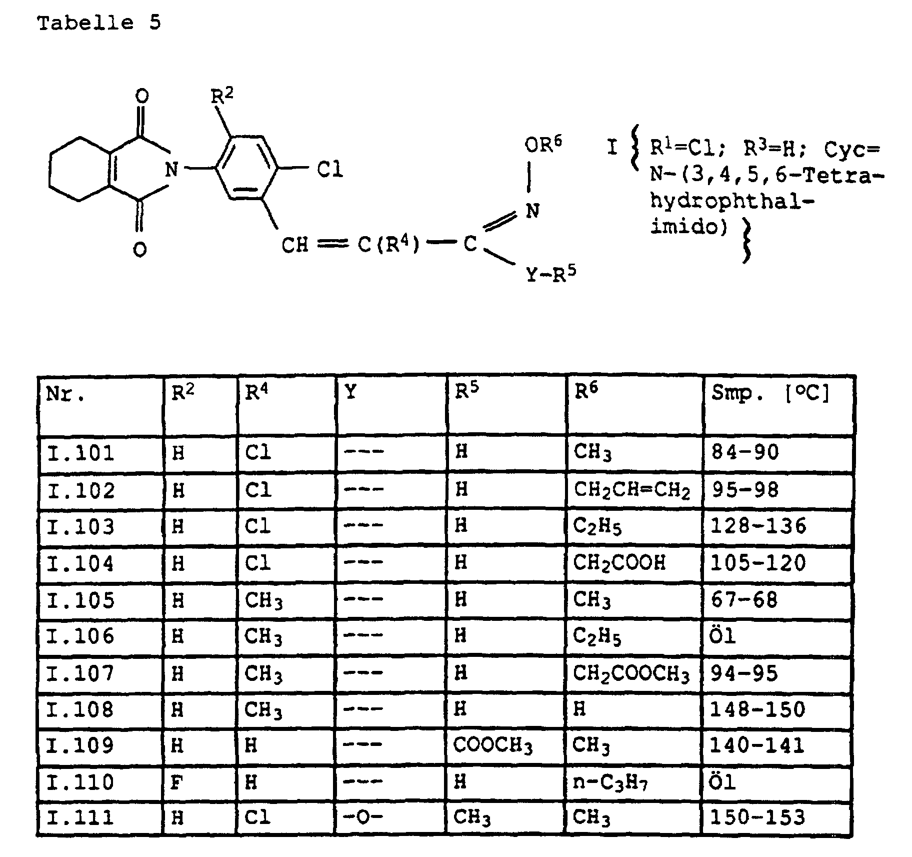

### Anwendungsbeispiele (herbizide Wirksamkeit)

Die herbizide Wirkung der substituierten Zimtoxim-Derivate und der substituierten Zimthydroxamid-Derivate der Formel II ließ sich durch Gewächshausversuche zeigen:

Als Kulturgefäße dienten Plastikblumentöpfe mit lehmigem Sand mit etwa 3,0% Humus als Substrat. Die Samen der Testpflanzen wurden nach Arten getrennt eingesät.

Bei Vorauf laufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,0313 oder 0,0156 kg aktive Substanz pro Hektar.

Die Pflanzen wurden artenspezifisch bei Temperaturen von 10 - 25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Ipomoea subspecies | Prunkwindearten | morningglory |
| Polygonum pensylvanicum | -------- | smartweed |
| Solanum nigrum | Schwarzer Nachtschatten | black nightshade |
| Veronica subspecies | Ehrenpreisarten | speedwell |

Im Nachauflaufverfahren wurden mit den Verbindungen Nr. 1.01 und 1.103 bei 0,0313 und 0,0156 kg/ha aktive Substanz Schadpflanzen sehr gut bekämpft.

Anwendungsbeispiele für die desiccative/defoliante Wirksamkeit der Verbindungen I und II:

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wäßrigen Aufbereitungen der Wirkstoffe (unter Zusatz von 0,15 Gew.-% des Fettalkoholalkoxylats Plurafac LF 700, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. Substituierte Zimtoxim-Derivate der Formel I 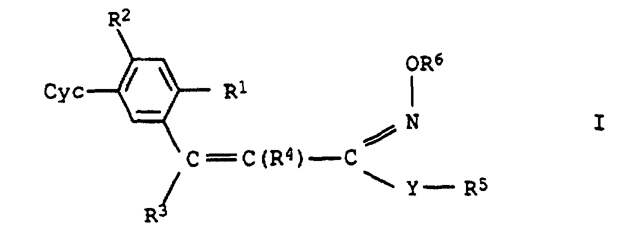 in der die Variablen folgende Bedeutung haben:
R¹ Halogen, Nitro, Cyano oder Trifluormethyl;
R² Wasserstoff oder Halogen;
R³ Wasserstoff, Halogen, C₁-C₆-Alkyl, C₁-C₆-Halogenalkyl, C₃-C₆-Cycloalkyl, C₃-C₆-Alkenyl, C₃-C₆-Alkinyl oder Hydroxy-C₁-C₆-alkyl;
R⁴ Wasserstoff, Halogen, Cyano, C₁-C₆-Alkyl, C₁-C₆-Halogenalkyl,
oder R³ und R⁴ zusammen eine chemische Bindung;
Y Sauerstoff; Schwefel, Oxycarbonyl, Oxysulfonyl oder eine chemische Bindung;
R⁵ C₁-C₆-Alkyl oder C₁-C₆-Halogenalkyl, wobei diese Gruppen unsubstituiert sein oder einen der folgenden Reste tragen können: Hydroxyl, Cyano, Hydroxycarbonyl, C₁-C₆-Alkoxyl, C₁-C₆-Alkylthio, (C₁-C₆-Alkyl)carbonyl, (C₁-C₆-Alkoxy)carbonyl, (C₁-C₆-Alkyl)carbonyloxy oder einen mit dem Stickstoffatom über eine Carbonylbrücke gebundenen 3- bis 7-gliedrigen Azaheterocyclus, der neben Kohlenstoffringgliedern noch ein Sauerstoff- oder Schwefelatom als Ringglied enthalten kann; C₃-C₆-Cycloalkyl, C₃-C₆-Alkenyl, C₃-C₆-Alkinyl, Phenyl oder Phenyl-C₁-C₆-alkyl, wobei die Phenylringe gewunschtenfalls ein bis drei Substituenten tragen können, ausgewahlt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
oder, sofern Y eine chemische Bindung bedeutet, zusätzlich Wasserstoff oder Halogen;
R⁶ Wasserstoff, C₁-C₆-Alkyl, C₁-C₆-Halogenalkyl, C₃-C₆-Cycloalkyl, C₃-C₆-Alkenyl, C₃-C₆-Alkinyl, Hydroxy-C₁-C₆-alkyl, C₁-C₆-Alkoxy-C₁-C₆-alkyl, C₁-C₆-Alkylthio-C₁-C₆-alkyl, Cyano-C₁-C₆-alkyl, (C₁-C₆-Alkoxy)carbonyl-C₁-C₆-alkyl, (C₁-C₆-Alkyl)carbonyl-C₁-C₆-alkyl, (C₁-C₆-Alkyl)carbonyloxy-C₁-C₆-alkyl oder Phenyl-C₁-C₆-alkyl, wobei der Phenylring gewünschtenfalls ein bis drei Substituenten tragen kann, ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl
oder, sofern Y Sauerstoff oder Schwefel bedeutet, R⁵ und R⁶ zusammen eine C₁-C₃-Alkylenkette, die einen C₁-C₆-Alkyl-Substituenten tragen kann;
Cyc N-(3,4,5,6-Tetrahydrophthalimido) oder einen Rest 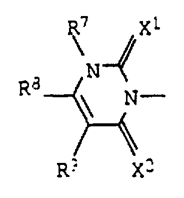 wobei X¹ und X² unabhangig voneinander für Sauerstoff oder Schwefel;
R⁷ für Wasserstoff, C₁-C₆-Alkyl, C₁-C₆-Halogenalkyl oder Amino und
R⁸ und R⁹ unabhängig voneinander fur Wasserstoff, Halogen, C₁-C₆-Alkyl, C₁-C₆-Halogenalkyl oder Phenyl, das gewünschtenfalls ein bis drei Substituenten tragen kann, ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl
stehen;
sowie die landwirtschaftlich brauchbaren Salze der Verbindungen I.

2. Substituierte Zimthydroxamid-Derivate der Formel II 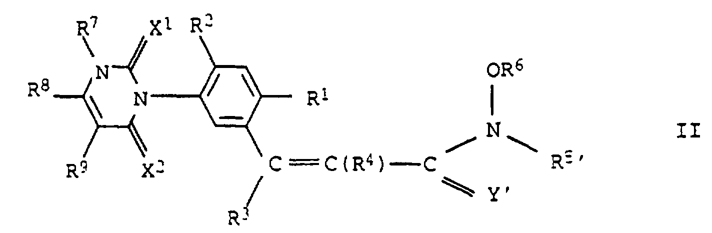 in der die Variablen folgende Bedeutung haben:
R¹ Halogen, Nitro, Cyano oder Trifluormethyl;
R² Wasserstoff oder Halogen;
R³ Wasserstoff, Halogen, C₁-C₆-Alkyl, C₁-C₆-Halogenalkyl, C₃-C₆-Cycloalkyl, C₃-C₆-Alkenyl, C₃-C₆-Alkinyl oder Hydroxy-C₁-C₆-alkyl;
R⁴ Wasserstoff, Halogen, Cyano, C₁-C₆-Alkyl, C₁-C₆-Halogenalkyl,
oder R³ und R⁴ zusammen eine chemische Bindung;
R⁵' Wasserstoff, C₁-C₆-Alkyl oder C₁-C₆-Halogenalkyl, wobei die letzten beiden Gruppen einen der folgenden Reste tragen können: Hydroxyl, Cyano, Hydroxycarbonyl, C₁-C₆-Alkoxy, C₁-C₆-Alkylthio, (C₁-C₆-Alkyl)carbonyl (C₁-C₆-Alkoxy)carbonyl, (C₁-C₆-Alkyl)carbonyloxy oder einen mit dem Stickstoffatom über eine Carbonylbrucke gebundenen 3- bis 7-gliedrigen Azaheterocyclus, der neben Kohlenstoffringgliedern noch ein Sauerstoff- oder Schwefelatom als Ringglied enthalten kann;
C₃-C₆-Cycloalkyl, C₃-C₆-Alkenyl, C₃-C₆-Alkinyl, (C₁-C₆-Alkyl)carbonyl, (C₁-C₆-Halogenalkyl)carbonyl (C₁-C₆-Alkoxy)carbonyl, Phenyl oder Phenyl-C₁-C₆-alkyl, wobei die Phenylringe gewunschtenfalls ein bis drei Substituenten tragen konnen, ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
R⁶ Wasserstoff, C₁-C₆-Alkyl, C₁-C₆-Halogenalkyl, C₃-C₆-Cycloalkyl, C₃-C₆-Alkenyl, C₃-C₆-Alkinyl, Hydroxy-C₁-C₆-alkyl, C₁-C₆-Alkoxy-C₁-C₆-alkyl, C₁-C₆-Alkylthio-C₁-C₆-alkyl, Cyano-C₁-C₆-alkyl, (C₁-C₆-Alkoxy)carbonyl-C₁-C₆-alkyl, (C₁-C₆-Alkyl)carbonyl-C₁-C₆-alkyl, (C₁-C₆-Alkyl)carbonyloxy-C₁-C₆-alkyl oder Phenyl-C₁-C₆-alkyl, wobei der Phenylring gewünschtenfalls ein bis drei Substituenten tragen kann, ausgewahlt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
Y', X¹ und X² unabhängig voneinander Sauerstoff oder Schwefel;
R⁷ Wasserstoff, C₁-C₆-Alkyl, C₁-C₆-Halogenalkyl oder Amino;
R⁸ und R⁹ unabhangig voneinander Wasserstoff, Halogen, C₁-C₆-Alkyl, C₁-C₆-Halogenalkyl oder Phenyl, das gewunschtenfalls ein bis drei Substituenten tragen kann, ausgewahlt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
sowie die landwirtschaftlich brauchbaren Salze der Verbindungen II.

3. Verwendung der substituierten Zimtoxim-Derivate der Formel I und der landwirtschaftlich brauchbaren Salze von I, gemäß Anspruch 1, als Herbizide oder zur Desikkacion und/oder Defoliation von Pflanzen.

4. Verwendung der substituierten Zimthydroxamid-Derivate der Formel II und der landwirtschaftlich brauchbaren Salze von II, gemäß Anspruch 2, als Herbizide oder zur Desikkation und/ oder Defoliation von Pflanzen.

5. Herbizides Mittel, enthaltend eine herbizid wirksame Menge mindestens eines substituierten Zimtoxim-Derivates der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvans.

6. Herbizides Mittel, enthaltend eine herbizid wirksame Menge mindestens eines substituierten Zimthydroxamid-Derivates der Formel II oder ein landwirtschaftlich brauchbares Salz von II, gemäß Anspruch 2, und mindestens einen inerten flüssigen und/oder festen Trägerstoff sowie gewünschtenfalls mindestens ein Adjuvans.

7. Mittel zur Desikkation und/oder Defoliation von Pflanzen, enthaltend eine desikkant und/oder defoliant wirksame Menge mindestens eines substituierten Zimtoxim-Derivates der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvans.

8. Mittel zur Desikkation und/oder Defoliation von Pflanzen, enthaltend eine desikkant und/oder defoliant wirksame Menge mindestens eines substituierten Zimthydroxamid-Derivates der Formel II oder ein landwirtschaftlich brauchbares Salz von II, gemäß Anspruch 2, und mindestens einen inerten flüssigen und/oder festen Trägerstoff sowie gewünschtenfalls mindestens ein Adjuvans.

9. Verfahren zur Herstellung von herbizid wirksamen Mitteln, **dadurch gekennzeichnet, daß** man eine herbizid wirksame Menge mindestens eines substituierten Zimtoxim-Derivates der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvans mischt.

10. Verfahren zur Herstellung von desikkant und/oder defoliant wirksamen Mitteln, **dadurch gekennzeichnet, daß** man eine desikkant/defoliant wirksame Menge mindestens eines substituierten Zimtoxim-Derivates der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvans mischt.

11. Verfahren zur Herstellung von herbizid wirksamen Mitteln, **dadurch gekennzeichnet, daß** man eine herbizid wirksame Menge mindestens eines substituierten Zimthydroxamid-Derivates der Formel II oder ein landwirtschaftlich brauchbares Salz von II, gemäß Anspruch 2, und mindestens einen inerten flüssigen und/oder festen Trägerstoff sowie gewünschtenfalls mindestens ein Adjuvans mischt.

12. Verfahren zur Herstellung von desikkant und/oder defoliant wirksamen Mitteln, **dadurch gekennzeichnet, daß** man eine desikkant/defoliant wirksame Menge mindestens eines substituierten Zimthydroxamid-Derivates der Formel II oder ein landwirtschaftlich brauchbares Salz von II, gemäß Anspruch 2, und mindestens einen inerten flüssigen und/oder festen Trägerstoff sowie gewünschtenfalls mindestens ein Adjuvans mischt.

13. Verfahren zur Bekämpfung von unerwünschtem Pflanzenwuchs, **dadurch gekennzeichnet, daß** man eine herbizid wirksame Menge mindestens eines substituierten Zimtoxim-Derivates der Formel I oder eines landwirtschaftlich brauchbaren Salzes von I, gemäß Anspruch 1, auf Pflanzen, deren Lebensraum oder auf Saatgut einwirken läßt.

14. Verfahren zur Desikkation und/oder Defoliation von Pflanzen, **dadurch gekennzeichnet, daß** man eine defoliant und/oder desikkant wirksame Menge mindestens eines substituierten Zimtoxim-Derivates der Formel I oder eines landwirtschaftlich brauchbaren Salzes von I, gemäß Anspruch 1, auf Pflanzen einwirken läßt.

15. Verfahren zur Bekämpfung von unerwünschtem Pflanzenwuchs, **dadurch gekennzeichnet, daß** man eine herbizid wirksame Menge mindestens eines substituierten Zimthydroxamid-Derivates der Formel II oder eines landwirtschaftlich brauchbaren Salzes von II, gemäß Anspruch 2, auf Pflanzen, deren Lebensraum oder auf Saatgut einwirken läßt.

16. Verfahren zur Desikkation und/oder Defoliation von Pflanzen, **dadurch gekennzeichnet, daß** man eine defoliant und/oder desikkant wirksame Menge mindestens eines substituierten Zimthydroxamid-Derivates der Formel II oder eines landwirtschaftlich brauchbaren Salzes von II, gemäß Anspruch 2, auf Pflanzen einwirken läßt.

17. Verfahren nach Anspruch 14, **dadurch gekennzeichnet, daß** man Baumwolle behandelt.

18. Verfahren nach Anspruch 16, **dadurch gekennzeichnet, daß** man Baumwolle behandelt.

19. Zimtoxime der Formel VIII 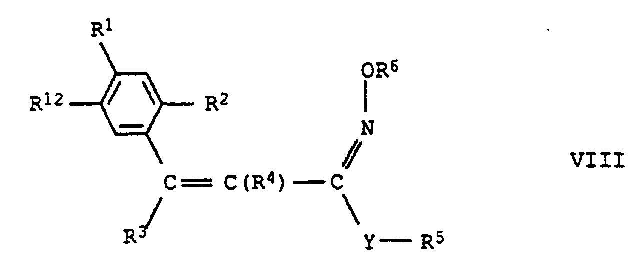 wobei R¹ bis R⁶ die entsprechenden Bedeutungen wie bei den Verbindungen I gemäß Anspruch 1 haben und wobei R¹² für Nitro, Amino, Isocyanato, Isothiocyanato, (C₁-C₆-Alkyl)carbamato oder Phenylcarbamato steht.

## Claims

1. A substituted cinnamic oxime derivative of the formula I 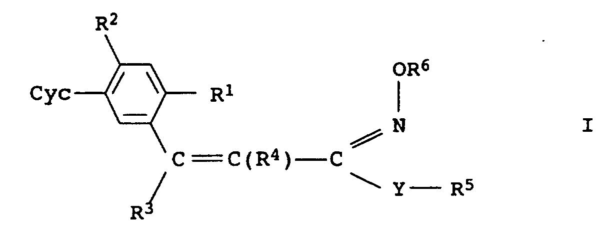 where the variables have the following meanings:
R¹ is halogen, nitro, cyano or trifluoromethyl;
R² is hydrogen or halogen;
R³ is hydrogen, halogen, C₁-C₆-alkyl, C₁-C₆-haloalkyl, C₃-C₆-cycloalkyl, C₃-C₆-alkenyl, C₃-C₆-alkynyl or hydroxy-C₁-C₆-alkyl;
R⁴ is hydrogen, halogen, cyano, C₁-C₆-alkyl, C₁-C₆-haloalkyl,
or R³ and R⁴ together are a chemical bond;
Y is oxygen, sulfur, oxycarbonyl, oxysulfonyl or a chemical bond;
R⁵ is C₁-C₆-alkyl or C₁-C₆-haloalkyl, it being possible for these groups to be unsubstituted or to carry one of the following radicals: hydroxyl, cyano, hydroxycarbonyl, C₁-C₆-alkoxy, C₁-C₆-alkylthio, (C₁-C₆-alkyl)carbonyl, (C₁-C₆-alkoxy)- carbonyl, (C₁-C₆-alkyl)carbonyloxy or a 3-to 7-membered azaheterocycle bonded to the nitrogen atom via a carbonyl bridge and which, in addition to carbon ring members, can also contain an oxygen or sulfur atom as a ring member;
is C₃-C₆-cycloalkyl, C₃-C₆-alkenyl, C₃-C₆-alkynyl, phenyl or phenyl-C₁-C₆-alkyl, it being possible for the phenyl rings, if desired, to carry one to three substituents selected from the group consisting of cyano, nitro, halogen, C₁-C₆-alkyl, C₁-C₆-haloalkyl, C₁-C₆-alkoxy and (C₁-C₆-alkoxy)carbonyl;
or, if Y is a chemical bond, is additionally hydrogen or halogen;
R⁶ is hydrogen, C₁-C₆-alkyl, C₁-C₆-haloalkyl, C₃-C₆-cycloalkyl, C₃-C₆-alkenyl, C₃-C₆-alkynyl, hydroxy-C₁-C₆-alkyl, C₁-C₆-alkoxy-C₁-C₆-alkyl, C₁-C₆-alkylthio-C₁-C₆-alkyl, cyano-C₁-C₆-alkyl, (C₁-C₆-alkoxy)carbonyl-C₁-C₆-alkyl,
(C₁-C₆-alkyl)carbonyl-C₁-C₆-alkyl, (C₁-C₆-alkyl)carbonyloxy-C₁-C₆-alkyl or phenyl-C₁-C₆-alkyl, it being possible for the phenyl ring, if desired, to carry one to three substituents selected from the group consisting of cyano, nitro, halogen, C₁-C₆-alkyl, C₁-C₆-haloalkyl, C₁-C₆-alkoxy and (C₁-C₆-alkoxy)carbonyl
or, if Y is oxygen or sulfur, R⁵ and R⁶ together are a C₁-C₃-alkylene chain which can carry a C₁-C₆-alkyl substituent;
Cyc is N-(3,4,5,6-tetrahydrophthalimido) or a radical 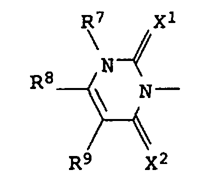
X¹ and X² independently of one another being oxygen or sulfur;
R⁷ being hydrogen, C₁-C₆-alkyl, C₁-C₆-haloalkyl or amino and
R⁸ and R⁹ independently of one another being hydrogen, halogen, C₁-C₆-alkyl, C₁-C₆-haloalkyl or phenyl which, if desired, can carry one to three substituents selected from the group consisting of cyano, nitro, halogen, C₁-C₆-alkyl, C₁-C₆-haloalkyl, C₁-C₆-alkoxy and (C₁-C₆-alkoxy)carbonyl;
and the agriculturally utilizable salts of the compounds I.

2. A substituted cinnamic hydroxamide derivative of the formula II 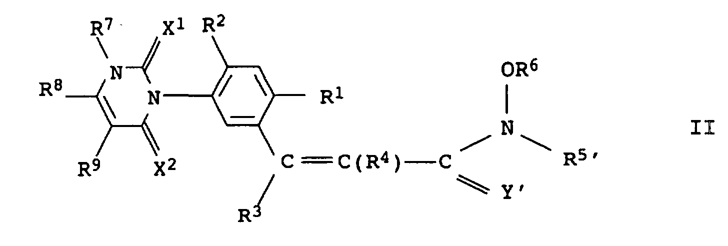 where the variables have the following meanings:
R¹ is halogen, nitro, cyano or trifluoromethyl;
R² is hydrogen or halogen;
R³ is hydrogen, halogen, C₁-C₆-alkyl, C₁-C₆-haloalkyl, C₃-C₆-cycloalkyl, C₃-C₆-alkenyl, C₃-C₆-alkynyl or hydroxy-C₁-C₆-alkyl;
R⁴ is hydrogen, halogen, cyano, C₁-C₆-alkyl, C₁-C₆-haloalkyl,
or R³ and R⁴ together are a chemical bond;
R⁵' is hydrogen, C₁-C₆-alkyl or C₁-C₆-haloalkyl, it being possible for the last two groups to carry one of the following radicals: hydroxyl, cyano, hydroxycarbonyl, C₁-C₆-alkoxy, C₁-C₆-alkylthio, (C₁-C₆-alkyl)carbonyl, (C₁-C₆-alkoxy)carbonyl, (C₁-C₆-alkyl)carbonyloxy or a 3- to 7-membered azaheterocycle bonded to the nitrogen atom via a carbonyl bridge and which, in addition to carbon ring members, can also contain an oxygen or sulfur atom as a ring member;
is C₃-C₆-cycloalkyl, C₃-C₆-alkenyl, C₃-C₆-alkynyl, (C₁-C₆-alkyl)carbonyl, (C₁-C₆-haloalkyl)carbonyl, (C₁-C₆-alkoxy)carbonyl, phenyl or phenyl-C₁-C₆-alkyl, it being possible for the phenyl rings, if desired, to carry one to three substituents selected from the group consisting of cyano, nitro, halogen, C₁-C₆-alkyl, C₁-C₆-haloalkyl, C₁-C₆-alkoxy and (C₁-C₆-alkoxy)carbonyl;
R⁶ is hydrogen, C₁-C₆-alkyl, C₁-C₆-haloalkyl, C₃-C₆-cycloalkyl, C₃-C₆-alkenyl, C₃-C₆-alkynyl, hydroxy-C₁-C₆-alkyl, C₁-C₆-alkoxy-C₁-C₆-alkyl, C₁-C₆-alkylthio-C₁-C₆-alkyl, cyano-C₁-C₆-alkyl, (C₁-C₆-alkoxy)carbonyl-C₁-C₆-alkyl, (C₁-C₆-alkyl)carbonyl-C₁-C₆-alkyl, (C₁-C₆-alkyl)carbonyloxy-C₁-C₆-alkyl or phenyl-C₁-C₆-alkyl, it being possible for the phenyl ring, if desired, to carry one to three substituents selected from the group consisting of cyano, nitro, halogen, C₁-C₆-alkyl, C₁-C₆-haloalkyl, C₁-C₆-alkoxy and (C₁-C₆-alkoxy)carbonyl;
Y', X¹ and X² independently of one another are oxygen or sulfur;
R⁷ is hydrogen, C₁-C₆-alkyl, C₁-C₆-haloalkyl or amino and
R⁸ and R⁹ independently of one another are hydrogen, halogen, C₁-C₆-alkyl, C₁-C₆-haloalkyl or phenyl which, if desired, can carry one to three substituents selected from the group consisting of cyano, nitro, halogen, C₁-C₆-alkyl, C₁-C₆-haloalkyl, C₁-C₆-alkoxy and (C₁-C₆-alkoxy)carbonyl;
and the agriculturally utilizable salts of the compounds II.

3. The use of the substituted cinnamic oxime derivatives of the formula I and the agriculturally utilizable salts of I, as claimed in claim 1, as herbicides or for the desiccation and/or defoliation of plants.

4. The use of the substituted cinnamic hydroxamide derivatives of the formula II and of the agriculturally utilizable salts of II, as claimed in claim 2, as herbicides or for the desiccation and/or defoliation of plants.

5. A herbicidal composition, containing a herbicidally active amount of at least one substituted cinnamic oxime derivative of the formula I or an agriculturally utilizable salt of I, as claimed in claim 1, and at least one inert liquid and/or solid carrier and also, if desired, at least one adjuvant.

6. A herbicidal composition, containing a herbicidally active amount of at least one substituted cinnamic hydroxamide derivative of the formula II or an agriculturally utilizable salt of II, as claimed in claim 2, and at least one inert liquid and/or solid carrier and also, if desired, at least one adjuvant.

7. A composition for the desiccation and/or defoliation of plants, containing an amount of at least one substituted cinnamic oxime derivative of the formula I or an agriculturally utilizable salt of I, as claimed in claim 1, having desiccant and/or defoliant activity and at least one inert liquid and/or solid carrier and also, if desired, at least one adjuvant.

8. A composition for the desiccation and/or defoliation of plants, containing an amount of at least one substituted cinnamic hydroxamide derivative of the formula II or an agriculturally utilizable salt of II, as claimed in claim 2, having desiccant and/or defoliant activity and at least one inert liquid and/or solid carrier and also, if desired, at least one adjuvant.

9. A process for preparing herbicidally active compositions, which comprises mixing a herbicidally active amount of at least one substituted cinnamic oxime derivative of the formula I or an agriculturally utilizable salt of I, as claimed in claim 1, and at least one inert liquid and/or solid carrier and also, if desired, at least one adjuvant.

10. A process for preparing compositions having desiccant and/or defoliant activity, which comprises mixing an amount of at least one substituted cinnamic oxime derivative of the formula I or an agriculturally utilizable salt of I, as claimed in claim 1, having defoliant/dessicant activity and at least one inert liquid and/or solid carrier and also, if desired, at least one adjuvant.

11. A process for preparing herbicidally active compositions, which comprises mixing a herbicidally active amount of at least one substituted cinnamic hydroxamide derivative of the formula II or an agriculturally utilizable salt of II, as claimed in claim 2, and at least one inert liquid and/or solid carrier and also, if desired, at least one adjuvant.

12. A process for preparing compositions having desiccant and/or defoliant activity, which comprises mixing an amount of at least one substituted cinnamic hydroxamide derivative of the formula II or an agriculturally utilizable salt of II, as claimed in claim 2, having a defoliant/dessicant activity and at least one inert liquid and/or solid carrier and also, if desired, at least one adjuvant.

13. A method of controlling undesired plant growth, which comprises allowing a herbicidally active amount of at least one substituted cinnamic oxime derivative of the formula I or of an agriculturally utilizable salt of I, as claimed in claim 1, to act on plants, their habitat or on seed.

14. A method for the desiccation and/or defoliation of plants, which comprises allowing an amount of at least one substituted cinnamic oxime derivative of the formula I or of an agriculturally utilizable salt of I, as claimed in claim 1, having defoliant and/or desiccant activity to act on plants.

15. A method of controlling undesired plant growth, which comprises allowing a herbicidally active amount of at least one substituted cinnamic hydroxamide derivative of the formula II or of an agriculturally utilizable salt of II, as claimed in claim 2, to act on plants, their habitat or on seed.

16. A method for the desiccation and/or defoliation of plants, which comprises allowing an amount of at least one substituted cinnamic hydroxamide derivative of the formula II or of an agriculturally utilizable salt of II, as claimed in claim 2, having defoliant and/or desiccant activity to act on plants.

17. A method as claimed in claim 14, wherein cotton is treated.

18. A method as claimed in claim 16, wherein cotton is treated.

19. A cinnamic oxime of the formula VIII 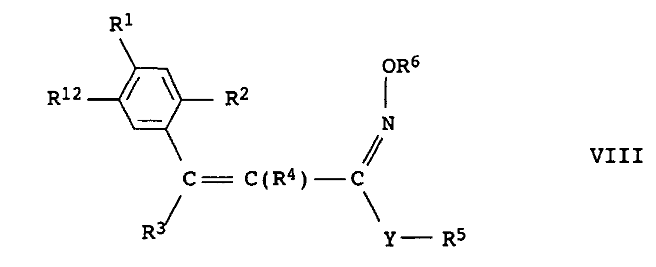 R¹ to R⁶ having the corresponding meanings to the compounds I as claimed in claim 1 and R¹² being nitro, amino, isocyanato, isothiocyanato, (C₁-C₆-alkyl)carbamato or phenylcarbamato.

## Revendications

1. Dérivés substitués de cinnamoximes, de formule I 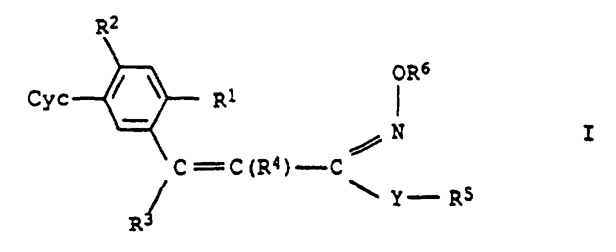 dans laquelle les symboles ont les significations suivantes :
R¹ : un halogène, un groupe nitro, cyano ou trifluorométhyle ;
R^{2 :} l'hydrogène ou un halogène,
R³ : l'hydrogène, un halogène, un groupe alkyle en C1-C6, halogénoalkyle en C1-C6, cycloalkyle en C3-C6, alcényle en C3-C6, alcynyle en C3-C6 ou hydroxyalkyle en C1-C6 ;
R⁴ : l'hydrogène, un halogène, un groupe cyano, alkyle en C1-C⁶, halogénoalkyle en C1-C6,
ou bien R³ et R⁴, ensemble, représentent une liaison chimique ;
Y : l'oxygène, le soufre, un groupe oxycarbonyle, oxysulfonyle ou une liaison chimique ;
R⁵ : un groupe alkyle en C1-C6 ou halogénoalkyle en C1-C6, ces groupes pouvant être non substitués ou pouvant porter un des substituants suivants : hydroxy, cyano, hydroxycarbonyle, alcoxy en C1-C6, alkylthio en C1-C6, (alkyle en C1-C6)carbonyle, (alcoxy en C1-C6)carbonyle, (alkyle en C1-C6)carbonyloxy ou un azahétérocycle de trois à sept chaînons relié à l'atome d'azote par l'intermédiaire d'un pont carbonyle et qui peut contenir, en plus des chaînons cycliques carbonés, un chaînon cyclique consistant en un atome d'oxygène ou un atome de soufre ;
un groupe cycloalkyle en C3-C6, alcényle en C3-C6, alcynyle en C3-C6, phényle ou phényl-alkyle en C3-C6, les cycles phényle pouvant porter le cas échéant un à trois substituants choisis parmi les groupes cyano, nitro, les halogènes, les groupes alkyle en C1-C6, halogénoalkyle en C1-C6, alcoxy en C1-C6 et (alcoxy en C1-C6)-carbonyle ;
ou bien encore, lorsque Y représente une liaison chimique, l'hydrogène ou un halogène ;
R⁶ : l'hydrogène, un groupe alkyle en C1-C6, halogénoalkyle en C1-C6, cycloalkyle en C3-C6, alcényle en C3-C6, alcynyle en C3-C6, hydroxyalkyle en C1-C6, (alcoxy en C1-C6)alkyle en C1-C6, (alkylthio en C1-C6)alkyle en C1-C6, cyano-alkyle en C1-C6, (alcoxy en C1-C6)carbonyl-alkyle en C1-C6, (alkyle en C1-C6)carbonyl-alkyle en C1-C6, (alkyle en C1-C6)carbonyloxy-alkyle en C1-C6 ou phényl-alkyle en C1-C6, le cycle phényle pouvant éventuellement porter un à trois substituants choisis parmi les groupes cyano, nitro, les halogènes, les groupes alkyle en C1-C6, halogénoalkyle en C1-C6, alcoxy en C1-C6 et (alcoxy en C1-C6)carbonyle,
ou bien encore, lorsque Y représente l'oxygène ou le soufre, R⁵ et R⁶ peuvent former ensemble une chaîne alkylène en C1-C3 qui peut porter un substituant alkyle en C1-C6 ;
Cyc : un groupe N-(3,4,5,6-tétrahydrophtalimido) ou un groupe 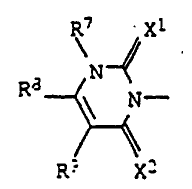
dans lequel X¹ et X² représentent chacun, indépendamment l'un de l'autre, l'oxygène ou le soufre ;
R⁷ : représente l'hydrogène, un groupe alkyle en C1-C6, halogénoalkyle en C1-C6 ou amino et
R⁸ et R⁹ représentent chacun, indépendamment l'un de l'autre, l'hydrogène, un halogène, un groupe alkyle en C1-C6, halogénoalkyle en C1-C6 ou phényle lequel peut lui-même porter un à trois substituants choisis parmi les groupes cyano, nitro, les halogènes, les groupes alkyle en C1-C6, halogénoalkyle en C1-C6, alcoxy en C1-C6 et (alcoxy en C1-C6)carbonyle,
et les sels des composés I aptes aux applications agricoles.

2. Dérivés substitués d'hydroxamides cinnamiques de formule II 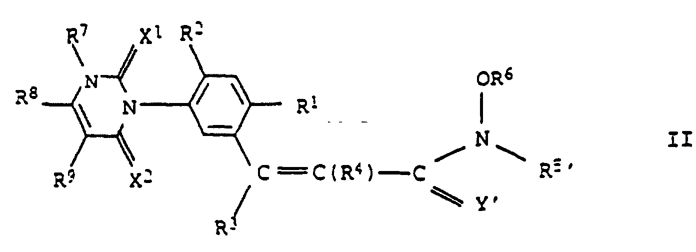 dans laquelle les symboles ont les significations suivantes :
R¹ : un halogène, un groupe nitro, cyano ou trifluorométhyle ;
R² : l'hydrogène ou un halogène ;
R³ : l'hydrogène, un halogène, un groupe alkyle en C1-C6, halogénoalkyle en C1-C6, cycloalkyle en C3-C6, alcényle en C3-C6, alcynyle en C3-C6 ou hydroxyalkyle en C1-C6 :
R⁴ : l'hydrogène, un halogène, un groupe cyano, alkyle en C1-C6, halogénoalkyle en C1-C6,
ou bien R³ et R⁴ forment ensemble une liaison chimique ;
R⁵ : l'hydrogène, un groupe alkyle en C1-C6 ou halogénoalkyle en C1-C6, ces deux derniers groupes pouvant porter l'un des substituants suivants: hydroxy, cyano, hydroxycarbonyle, alcoxy en C1-C6, alkylthio en C1-C6, (alkyle en C1-C6)carbonyle, (alcoxy en C1-C6)carbonyle, (alkyle en C1-C6)carbonyloxy ou un azahétérocycle de trois à sept chaînons relié à l'atome d'azote par l'intermédiaire d'un pont carbonyle qui, en plus des chaînons cycliques carbonés, peut contenir un chaînon cyclique consistant en un atome d'oxygène ou un atome de soufre ;
un groupe cycloalkyle en C3-C6, alcényle en C3-C6, alcynyle en C3-C6, (alkyle en C1-C6)carbonyle, (halogénoalkyle en C1-C6)carbonyle, (alcoxy en C1-C6)carbonyle, phényle ou phényl-alkyle en C1-C4, les cycles phényle pouvant le cas échéant porter un à trois substituants choisis parmi les groupes cyano, nitro, les halogènes, les groupes alkyle en C1-C6, halogénoalkyle en C1-C6, alcoxy en C1-C6 et (alcoxy en C1-C6)carbonyle ;
R⁶ : l'hydrogène, un groupe alkyle en C1-C6, halogénoalkyle en C1-C6, cycloalkyle en C3-C6, alcényle en C3-C6, alcynyle en C3-C6, hydroxyalkyle en C1-C6, (alcoxy en C1-C6)alkyle en C1-C6, (alkylthio en C1-C6)alkyle en C1-C6, cyano-alkyle en C1-C6, (alcoxy en C1-C6)carbonyl-alkyle en C1-C6, (alkyle en C1-C6)carbonyl-alkyle en C1-C6), (alkyle en C1-C6)carbonyloxy-alkyle en C1-C6 ou phényl-alkyle en C1-C6, le cycle phényle pouvant lui-même porter un à trois substituants choisis parmi les groupes cyano, nitro, les halogènes, les groupes alkyle en C1-C6, halogénoalkyle en C1-C6, alcoxy en C1-C6 et (alcoxy en C1-C6)carbonyle ;
Y', X¹ et X² représentent chacun, indépendamment les uns des autres, l'oxygène ou le soufre ;
R⁷ : représente l'hydrogène, un groupe alkyle en C1-C6, halogénoalkyle en C1-C6 ou amino ;
R⁸ et R⁹ représentent chacun, indépendamment l'un de l'autre, l'hydrogène, un halogène, un groupe alkyle en C1-C6, halogénoalkyle en C1-C6 ou phényle, lequel peut porter un à trois substituants choisis parmi les groupes cyano, nitro, les halogènes, les groupes alkyle en C1-C6, halogénoalkyle en C1-C6, alcoxy en C1-C6 et (alcoxy en C1-C6)carbonyle ;
et les sels des composés II aptes aux applications agricoles.

3. Utilisation des dérivés substitués de cinnamoximes de formule I et des sels de I aptes aux applications agricoles selon la revendication 1 en tant que produits herbicides ou produits pour la dessiccation et/ou la défoliation de végétaux.

4. Utilisation des dérivés substitués d'hydroxamides cinnamiques de formule II et des sels de II aptes aux applications agricoles selon la revendication 2 en tant que produits herbicides ou produits pour la dessiccation et/ou la défoliation des végétaux.

5. Produit herbicide contenant une quantité herbicide efficace d'au moins un dérivé substitué de cinnamoxime de formule I ou d'un sel de I apte aux applications agricoles selon la revendication 1 et au moins un véhicule inerte liquide et/ou solide et le cas échéant au moins un adjuvant.

6. Produit herbicide contenant une quantité herbicide efficace d'au moins un dérivé substitué d'hydroxamide cinnamique de formule II ou d'un sel de II apte aux applications agricoles selon la revendication 2 et au moins un véhicule inerte liquide et/ou solide avec, le cas échéant, au moins un adjuvant.

7. Produit pour la dessiccation et/ou la défoliation de végétaux, contenant une quantité dessiccante et/ou défoliante efficace d'au moins un dérivé substitué de cinnamoxime de formule I ou d'un sel de I apte aux applications agricoles selon la revendication 1, et au moins un véhicule inerte liquide et/ou solide avec, le cas échéant, au moins un adjuvant.

8. Produit pour la dessiccation et/ou la défoliation de végétaux, contenant une quantité dessiccante et/ou défoliante efficace d'au moins un dérivé substitué d'hydroxamide cinnamique de formule II ou d'un sel de II apte aux applications agricoles selon la revendication 2 et au moins un véhicule inerte liquide et/ou solide avec, le cas échéant, au moins un adjuvant.

9. Procédé pour préparer des produits à activité herbicide, **caractérisé par le fait que** l'on mélange une quantité herbicide efficace d'au moins un dérivé substitué de cinnamoxime de formule I ou d'un sel de I apte aux applications agricoles selon la revendication 1 et au moins un véhicule inerte liquide et/ou solide avec, le cas échéant, au moins un adjuvant.

10. Procédé pour préparer des produits à activité dessiccante et/ou défoliante, **caractérisé par le fait que** l'on mélange une quantité dessiccante/défoliante efficace d'au moins un dérivé substitué de cinnamoxime de formule I ou d'un sel de I apte aux applications agricoles selon la revendication 1 et au moins un véhicule inerte liquide et/ou solide avec, le cas échéant, au moins un adjuvant.

11. Procédé pour la préparation de produits à activité herbicide, **caractérisé par le fait que** l'on mélange une quantité herbicide efficace d'au moins un dérivé substitué d'hydroxamide cinnamique de formule I ou d'un sel de I apte aux applications agricoles selon la revendication 1, et au moins un véhicule inerte solide et/ou liquide avec, le cas échéant, au moins un adjuvant.

12. Procédé pour la préparation de produits à activité dessiccante et/ou défoliante, **caractérisé par le fait que** l'on mélange une quantité dessiccante/défoliante efficace d'au moins un dérivé substitué d'hydroxamide cinnamique de formule II ou d'un sel de II apte aux applications agricoles selon la revendication 2 et au moins un véhicule inerte liquide et/ou solide avec, le cas échéant, au moins un adjuvant.

13. Procédé pour combattre les croissances de végétaux indésirables, **caractérisé par le fait que** l'on fait agir une quantité herbicide efficace d'au moins un dérivé substitué de cinnamoxime de formule I ou d'un sel de I apte aux applications agricoles selon la revendication 1 sur les végétaux, leur habitat ou sur les semences.

14. Procédé pour la dessiccation et/ou la défoliation de végétaux, **caractérisé par le fait que** l'on fait agir une quantité dessiccante et/ou défoliante efficace d'au moins un dérivé substitué de cinnamoxime de formule I ou d'un sel de I apte aux applications agricoles selon la revendication 1 sur les végétaux.

15. Procédé pour combattre les croissances de végétaux indésirables, **caractérisé par le fait que** l'on fait agir une quantité herbicide efficace d'au moins un dérivé substitué d'hydroxamide cinnamique de formule II ou d'un sel de II apte aux applications agricoles selon la revendication 2 sur les végétaux, leur habitat ou sur les semences.

16. Procédé pour la dessiccation et/ou la défoliation de végétaux, **caractérisé par le fait que** l'on fait agir une quantité défoliante et/ou dessiccante efficace d'au moins un dérivé substitué d'hydroxamide cinnamique de formule II ou d'un sel de II apte aux applications agricoles selon la revendication 2 sur les végétaux.

17. Procédé selon la revendication 14, **caractérisé par le fait que** l'on traite du coton.

18. Procédé selon la revendication 16, **caractérisé par le fait que** l'on traite du coton.

19. Cinnamoximes de formule VIII 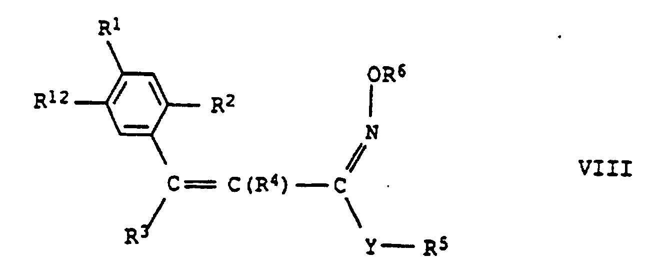 dans laquelle R¹ à R⁶ ont les significations correspondant à celles indiquées pour les composés I selon la revendication 1, et R¹² représente un groupe nitro, amino, isocyanato, isothiocyanato, (alkyle en C1-C6)carbamato ou phénylcarbamato.
